# EUROPEAN PATENT SPECIFICATION

(11) **EP 0 811 611 B1**
(45) Date of publication and mention of the grant of the patent: **26.01.2000**
(21) Application number: 97109375.2
(22) Date of filing: 13.05.1994
(51) Int. Cl.: C07C 311/08, C07C 311/14, C07C 311/21, A01N 37/34

(54) **2-cyano-1-sulfonamidophenyl-1,3-dione derivatives and their use as herbicides**
2-Cyan-1-sulfonamidophenyl-1,3-dion-Derivate und deren Verwendung als Herbizide
Dérivés de 2-cyano-1-sulfonamidophényl-1,3-dione et leur emploi comme herbicides

(30) Priority: 18.05.1993 GB 9310222; 22.07.1993 US 94881
(43) Date of publication of application: 10.12.1997
(62) Divisional of application: 94107449.4
(73) Proprietor: RHONE-POULENC AGRICULTURE LTD., Ongar, Essex CM5 0HW (GB)
(72) Inventor: Cramp, Susan Mary, Ongar, Essex, CM5 0HW (GB); Pettit, Simon Neil, Ongar, Essex, CM5 0HW (GB); Musil, Tibor, 64060 Akers, Styckebruk (SE); Smith, Philip Henry Gaunt, Essex, CM5 0HW (GB)
(74) Representative: Brachotte, Charles

(56) References cited:
- EP-A- 0 213 892
- EP-A- 0 496 630
- EP-A- 0 496 631

## Description

This invention relates to novel 2-cyano-1,3-dione derivatives, compositions containing them, processes for their preparation and their use as herbicides.

2-Cyano-1,3-diones are disclosed in Chemical Abstracts, Vol. 100 (1984), 157636t; Chemical Abstracts Vol. 93(1980), 178628u; Bull. Chem. Soc. Jpn., Vol. 44(1) (1971), pp185 - 189; Ind. J. Chem., Vol. 25 (1986), pp1133 - 1137; J Het Chem., Vol. 20 (1983), pp645 - 648; and BE-A-819136. Herbicidal 2-cyano-1,3-diones are described in EP-A-0213892, -0496630 and -0496631.

The present invention provides 2-cyano-1,3-dione derivatives of formula (I): 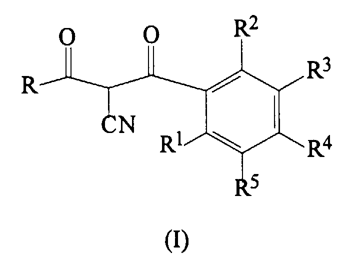 wherein:
R represents:-
   a straight- or branched- chain alkyl group containing up to six carbon atoms which is optionally substituted by one or more halogen atoms; or
   a cycloalkyl group containing from three to six carbon atoms optionally substituted by one or more R⁷ groups;
R¹ represents the hydrogen atom;
R², R³, R⁴ and R⁵, which may be the same or different, each represents:-
   the hydrogen atom;
   a straight- or branched- chain alkyl, alkenyl or alkynyl group containing up to six carbon atoms optionally substituted by one or more halogen atoms;
   a straight- or branched- chain alkyl group containing up to six carbon atoms which is substituted by a group -OR⁶;
   a halogen atom;
   phenyl optionally substituted by from one to three groups R²¹ which may be the same or different;
   a group selected from -COR⁷, nitro, cyano, -CO₂R⁶, -S(O)ₙR⁹, -O(CH₂)ₘOR⁶, -N(R¹²)SO₂R⁸, -CONR¹⁰R¹⁵ and -OR⁶¹;
   provided that at least one of the groups R² to R⁵ represents -N(R¹²)SO₂R⁸;
R⁶ represents:-
   a straight- or branched- chain alkyl, alkenyl or alkynyl group containing up to six carbon atoms optionally substituted by one or more halogen atoms; or
   a cycloalkyl group containing from three to six carbon atoms;
R⁶¹ represents:-
   a straight- or branched- chain alkyl, alkenyl or alkynyl group containing up to six carbon atoms optionally substituted by one or more halogen atoms;
   a cycloalkyl group containing from three to six carbon atoms; or
   phenyl optionally substituted by from one to five groups R²¹ which may be the same or different;
R⁷ represents:-
   a straight- or branched- chain alkyl group containing up to six carbon atoms optionally substituted by one or more halogen atoms;
   or a cycloalkyl group containing from three to six carbon atoms;
R⁸ represents:-
   a straight- or branched- chain alkyl, alkenyl or alkynyl group containing up to six carbon atoms optionally substituted by one or more halogen atoms;
   a cycloalkyl group containing from three to six carbon atoms;
   phenyl optionally substituted by from one to five groups R²¹ which may be the same or different; or ⁻NR¹⁰R¹¹;
R⁹ represents:-
   a straight- or branched- chain alkyl, alkenyl or alkynyl group containing up to six carbon atoms optionally substituted by one or more halogen atoms;
   a cycloalkyl group containing from three to six carbon atoms;
   or phenyl optionally substituted by from one to five groups R²¹ which may be the same or different;
R¹⁰ represents hydrogen or a straight- or branched- chain alkyl group containing up to six carbon atoms optionally substituted by one or more halogen atoms;
R¹¹ represents:-
   a straight- or branched- chain alkyl group containing up to six carbon atoms optionally substituted by one or more halogen atoms;
   where R¹⁰ and R¹¹ are part of a group -NR¹⁰R¹¹ they may, together with the nitrogen to which they are attached, form a five or six membered ring optionally having one additional heteroatom in the ring which is oxygen or nitrogen (e.g. pyrrolidine, morpholine, pyrrole, piperidine and piperazine), wherein the ring is optionally substituted by one or more alkyl groups containing up to three carbon atoms;
R¹² represents:
   the hydrogen atom; or
   a straight- or branched- chain alkyl, alkenyl or alkynyl group containing up to six carbon atoms optionally substituted by one or more halogen atoms;
   a cycloalkyl group containing from three to six carbon atoms;
   phenyl optionally substituted by from one to five groups R²¹ which may be the same or different; or
   a group -OR¹⁷
R¹⁵ represents a group selected from R⁷ and -OR¹⁷;
where R¹⁰ and R¹⁵ are part of a group -CONR¹⁰R¹⁵ they may, together with the nitrogen to which they are attached, form a 5 or 6 membered ring optionally having one additional heteroatom in the ring which is oxygen or nitrogen (e.g. pyrrolidine, morpholine, pyrrole, piperidine and piperazine), wherein the ring is optionally substituted by one or more alkyl groups containing up to 3 carbon atoms;
R¹⁷ represents a straight- or branched- chain alkyl group containing up to six carbon atoms;
R²¹ represents
   a halogen atom;
   a straight- or branched- chain alkyl group containing up to three carbon atoms optionally substituted by one or more halogen atoms;
   or a group selected from nitro, cyano, -S(O)ₙR⁷ and -OR⁷;
   m represents one, two or three; and
   n represents zero, one or two;
   and agriculturally acceptable salts or metal complexes thereof, which possess valuable properties.

It will be understood that the compounds may exist in enolic tautomeric forms. All such forms are embraced by the present invention.

Compounds of formula I may exist in enolic tautomeric forms that may give rise to geometric isomers around the enolic double bond.

Furthermore in certain cases the groups R¹, R², R³, R⁴ and R⁵ may give rise to stereoisomers and geometric isomers. All such forms are embraced by the present invention.

By the term "agriculturally acceptable salts" is meant salts the cations or anions of which are known and accepted in the art for the formation of salts for agricultural or horticultural use. Preferably the salts are water-soluble. Suitable salts with bases include alkali metal (eg. sodium and potassium), alkaline earth metal (eg. calcium and magnesium), ammonium and amine (eg. diethanolamine, triethanolamine, octylamine, morpholine and dioctylmethylamine) salts. Suitable acid addition salts, formed by compounds of formula (I) containing an amino group, include salts with inorganic acids, for example hydrochlorides, sulphates, phosphates and nitrates and salts with organic acids, for example acetic acid.

By the term "metal complexes" is meant compounds in which one or both of the oxygen atoms of the 1,3-dione act as chelating agents to a metal cation. Examples of such cations include zinc, manganese, cupric, cuprous, ferric, ferrous, titanium and aluminium.

The compounds of the invention, in some aspects of their activity, show advantages over known compounds.

A preferred class of compounds of formula (I) are those wherein:
R represents:-
   a straight- or branched- chain alkyl group containing up to six carbon atoms which is optionally substituted by one or more halogen atoms; or
   a cycloalkyl group containing from three to six carbon atoms optionally substituted by one or more methyl groups;
R², R³, R⁴ and R⁵, which may be the same or different, each represents:-
   a hydrogen or halogen atom;
   a straight- or branched- chain alkyl, alkenyl or alkynyl group containing up to six carbon atoms optionally substituted by one or more halogen atoms;
   a straight- or branched- chain alkyl group containing up to six carbon atoms which is substituted by a group -OR⁶;
   phenyl optionally substituted by from one to three groups R²¹ which may be the same or different;
   or a group selected from -COR⁷, cyano, nitro, -CO₂R⁶, -S(O)ₙR⁹, -O(CH₂)ₘOR⁶, -N(R¹²)SO₂R⁸ and -OR⁶¹;
R⁶ and R⁷ which may be the same or different, each represents:-
   a straight- or branched- chain alkyl group containing up to six carbon atoms optionally substituted by one or more halogen atoms;
   or a cycloalkyl group containing from three to six carbon atoms;
R⁶¹ and R⁹, which may be the same or different, each represents:-
   a straight- or branched- chain alkyl or alkenyl group containing up to six carbon atoms optionally substituted by one or more halogen atoms;
   a straight- or branched- chain alkynyl group containing from three to six carbon atoms;
   a cycloalkyl group containing three to six carbon atoms;
R⁸ represents:-
   a straight- or branched- chain alkyl or alkenyl group containing up to six carbon atoms optionally substituted by one or more halogen atoms;
   a straight- or branched- chain alkynyl group containing from three to six carbon atoms;
   a cycloalkyl group containing three to six carbon atoms; or
   phenyl optionally substituted by from one to three groups R²¹ which may be the same or different;
R¹² represents:-
   the hydrogen atom; or
   a straight- or branched- chain alkyl or alkenyl group containing up to six carbon atoms optionally substituted by one or more halogen atoms;
   a straight- or branched- chain alkynyl group containing from three to six carbon atoms;
   or a cycloalkyl group containing three to six carbon atoms;
R²¹ represents:-
   a halogen atom;
   a straight- or branched- chain alkyl group containing up to three carbon atoms optionally substituted by one or more halogen atoms; or
   a group selected from nitro, cyano, -S(O)ₙR⁷ or -OR⁷;
   m represents two or three; and
   n represents zero, one or two.

A further preferred class of compounds of formula (I) consists of those wherein:-
R represents:-
   a straight- or branched- chain alkyl group containing up to three carbon atoms; or
   a cycloalkyl group containing three or four carbon atoms optionally substituted by a methyl group;
R², R³ and R⁴ which may be the same or different, each represents:-
   a hydrogen, chlorine, bromine or fluorine atom; or
   a straight- or branched- chain alkyl, alkenyl or alkynyl group containing up to four carbon atoms optionally substituted by one or more halogen atoms;
   a straight- or branched- chain alkyl group containing up to four carbon atoms which is substituted by a group -OR⁶; or
   a group selected from -COR⁷, -CO₂R⁶, -S(O)ₙR⁹, -O(CH₂)ₘOR⁶, -N(R¹²)SO₂R⁸ and -OR⁶¹;
R⁵ represents the hydrogen atom;
R⁶, R⁷, and R⁹ which may be the same or different, each represents:-
   a straight- or branched- chain alkyl group containing up to four carbon atoms optionally substituted by one or more chlorine, bromine or fluorine atoms; or
   a cyclopropyl group;
R⁶¹ and R⁸, which may be the same or different, each represents:-
   a straight- or branched- chain alkyl or alkenyl group containing up to four carbon atoms optionally substituted by one or more chlorine, bromine or fluorine atoms;
   a straight- or branched- chain alkynyl group containing three or four carbon atoms; or
   a cyclopropyl group;
R¹² represents:-
   the hydrogen atom;
   a straight- or branched- chain alkyl or alkenyl group containing up to four carbon atoms optionally substituted by one or more chlorine, bromine or fluorine atoms;
   a straight- or branched- chain alkynyl group containing three or four carbon atoms; or
   a cyclopropyl group;
   m represents two or three; and
   n represents zero, one or two.

A further preferred class of compounds of formula (I) consists of those wherein:-
R represents a methyl, ethyl, isopropyl, cyclopropyl or 1-methylcyclopropyl group;
R², R³ and R⁴ which may be the same or different, each represents:-
   a hydrogen, bromine, chlorine or fluorine atom; or
   a straight- or branched- chain alkyl or alkenyl group containing up to four carbon atoms optionally substituted by one or more chlorine, bromine or fluorine atoms;
   a group selected from -COR⁷, -CO₂R⁶, -SR⁹-, -O(CH₂)ₘOR⁶, -OR⁶¹ and -N(R¹²)SO₂R⁸; or
   a straight- or branched chain alkyl group containing up to four carbon atoms which is substituted by -OR⁶;
R⁵ represents the hydrogen atom;
R⁶, R⁷ and R⁹ which may be the same or different, each represents a straight- or branched- chain alkyl group containing up to three carbon atoms;
R⁶¹ represents:-
   a straight- or branched- chain alkyl group containing up to four carbon atoms optionally substituted by one or more chlorine, bromine or fluorine atoms;
   a straight- or branched- chain alkenyl or alkynyl group containing three or four carbon atoms; or
   a cyclopropyl group;
R⁸ represents:-
   a straight- or branched- chain alkyl group containing up to three carbon atoms optionally substituted by one or more chlorine, bromine, or fluorine atoms; or
   an allyl group optionally substituted by one or more chlorine, fluorine or bromine atoms;
R¹² represents:-
   the hydrogen atom;
   a straight- or branched- chain alkyl group containing up to three carbon atoms optionally substituted by one or more chlorine, bromine, or fluorine atoms; or
   an allyl group optionally substituted by one or more chlorine, fluorine or bromine atoms;
   m represents two or three.

Examples of specific compounds embraced by formula (I) include the following:

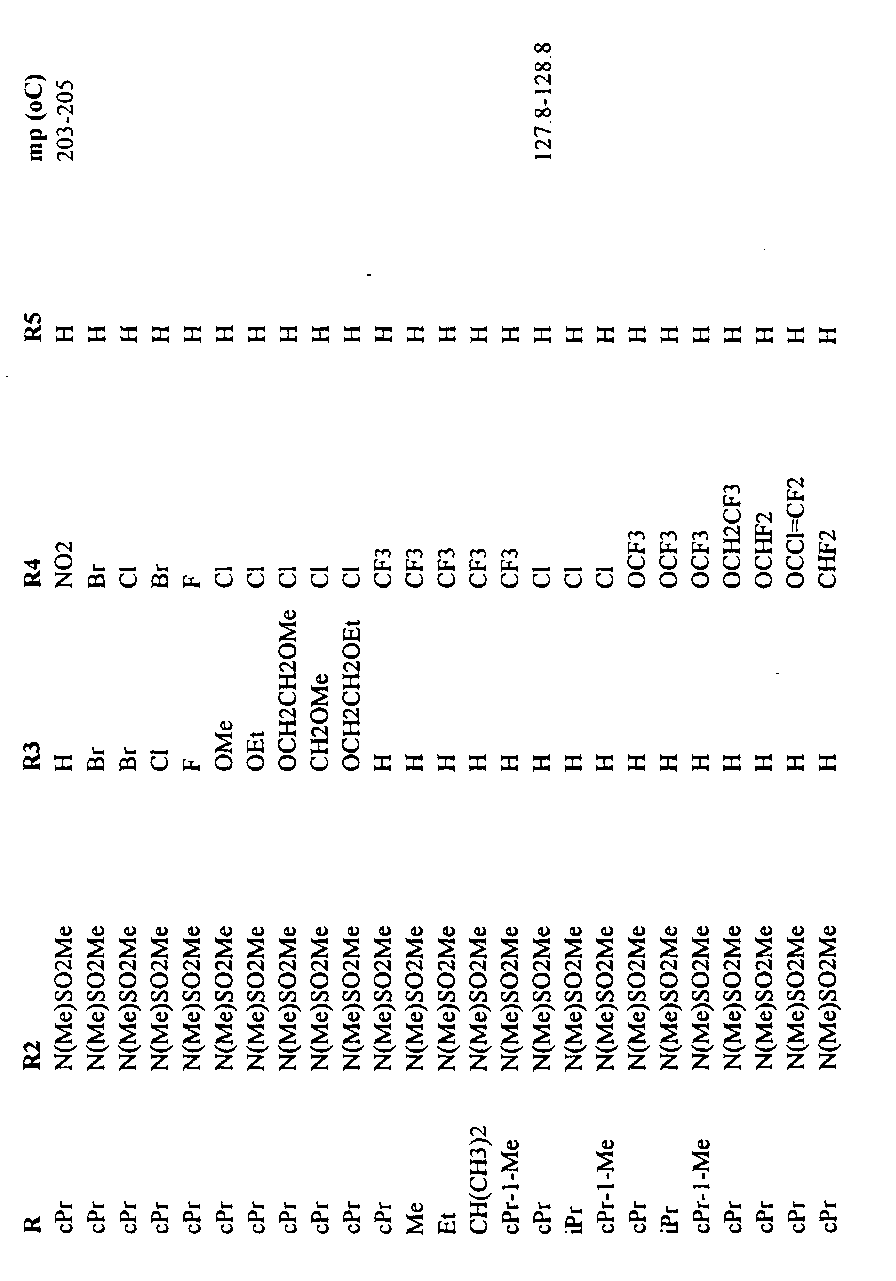

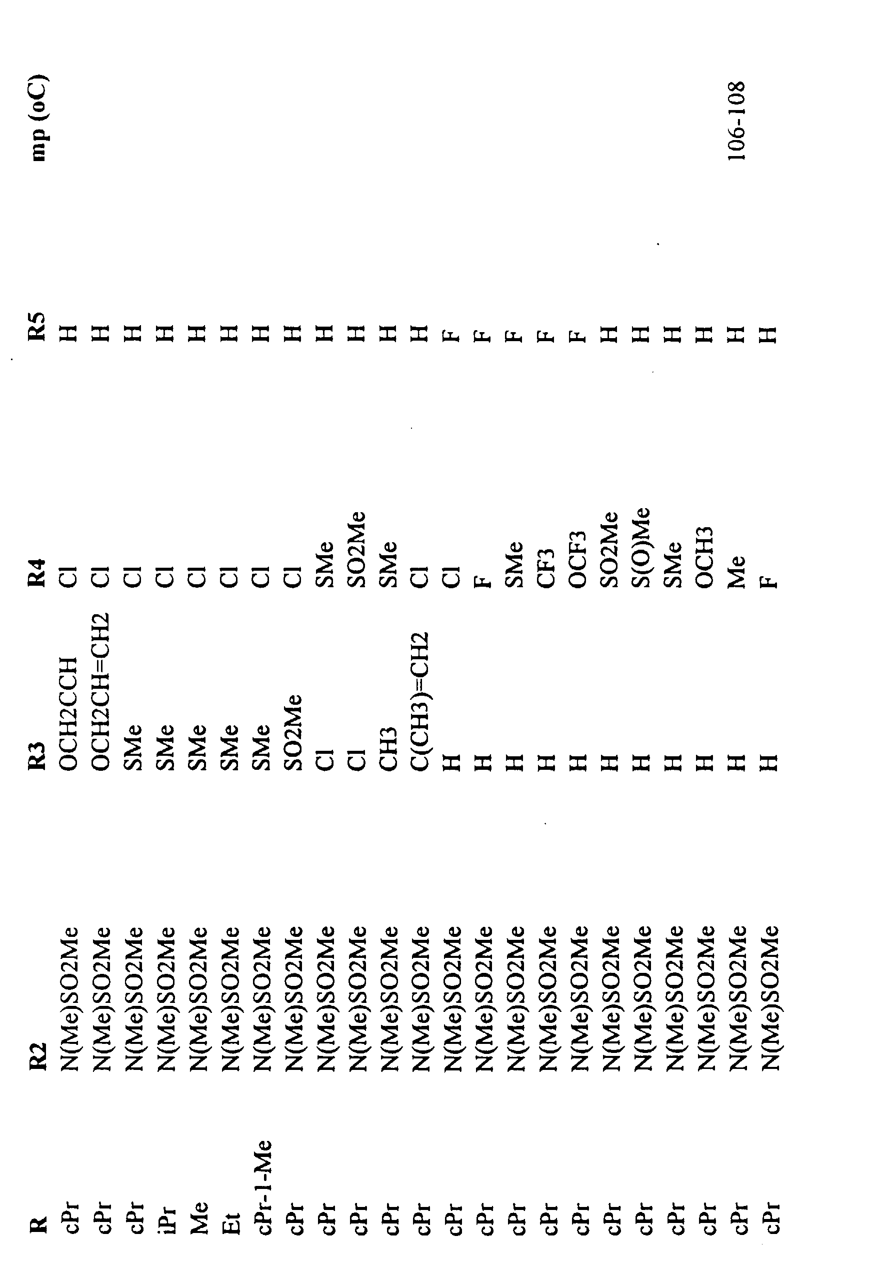

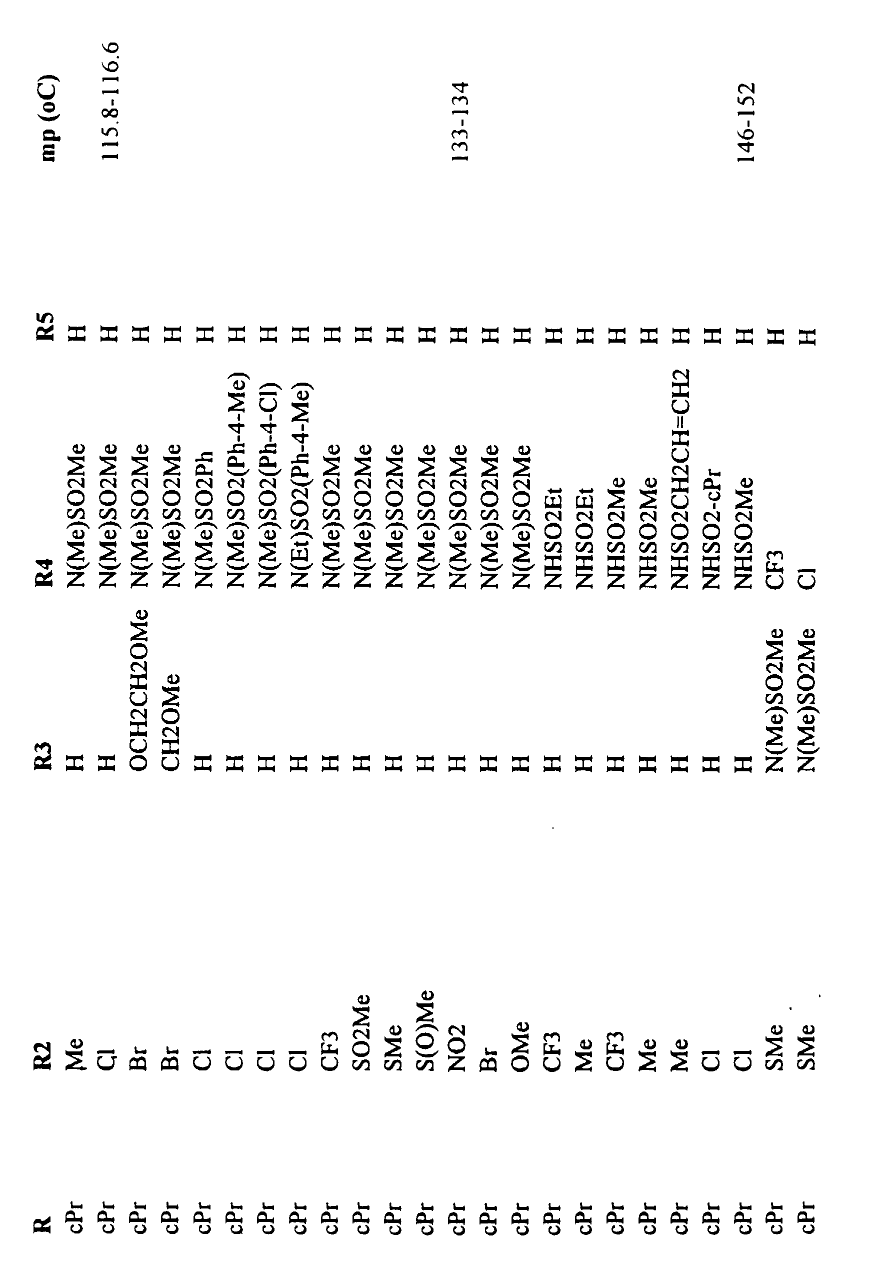

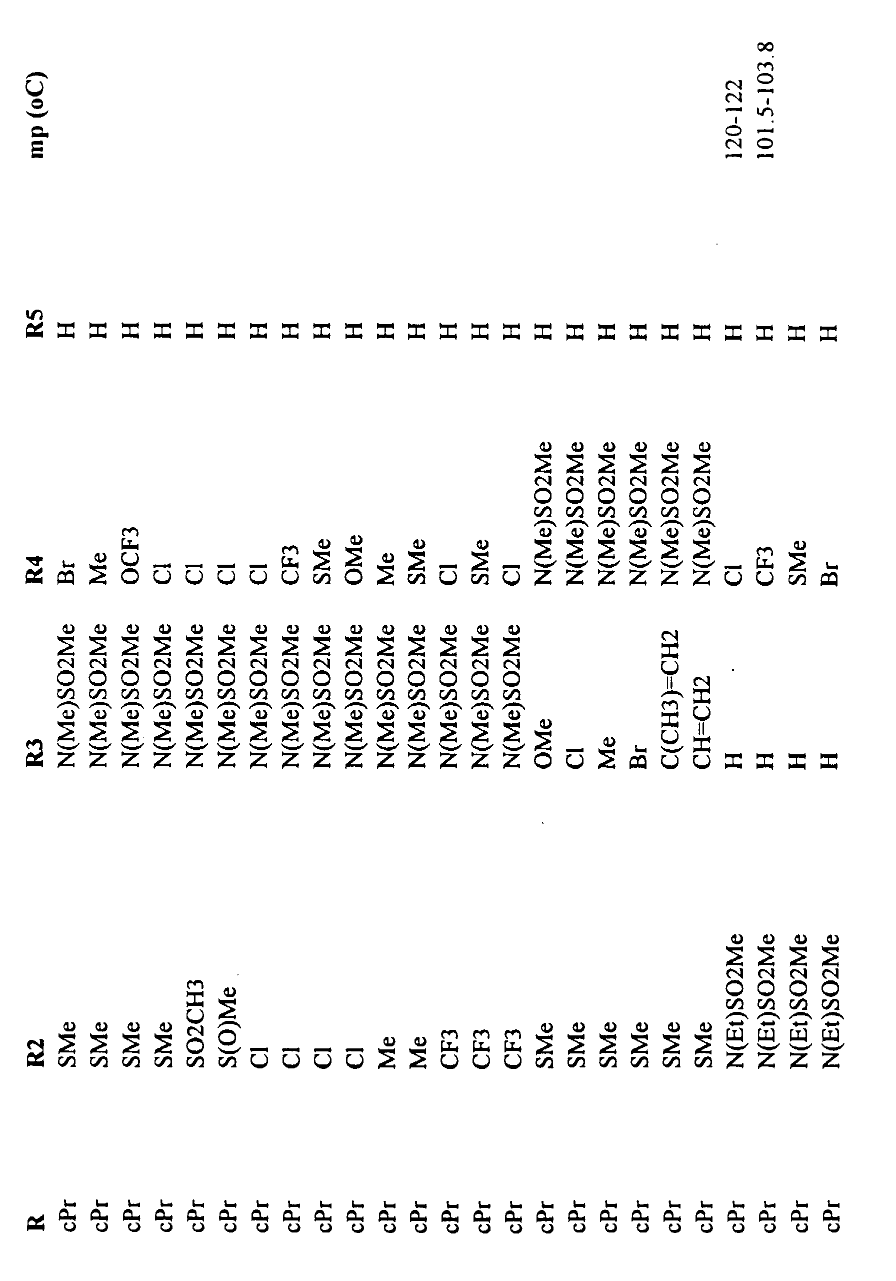

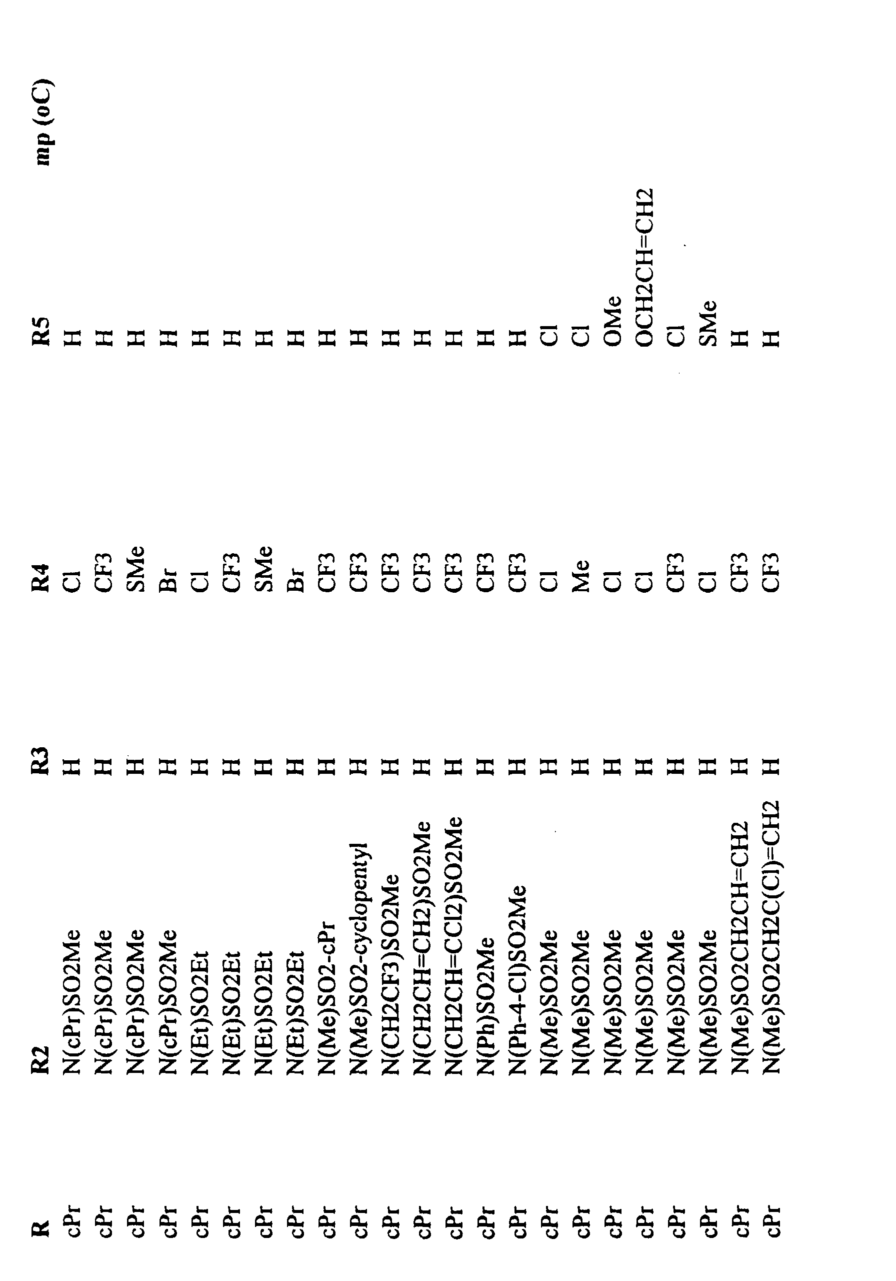

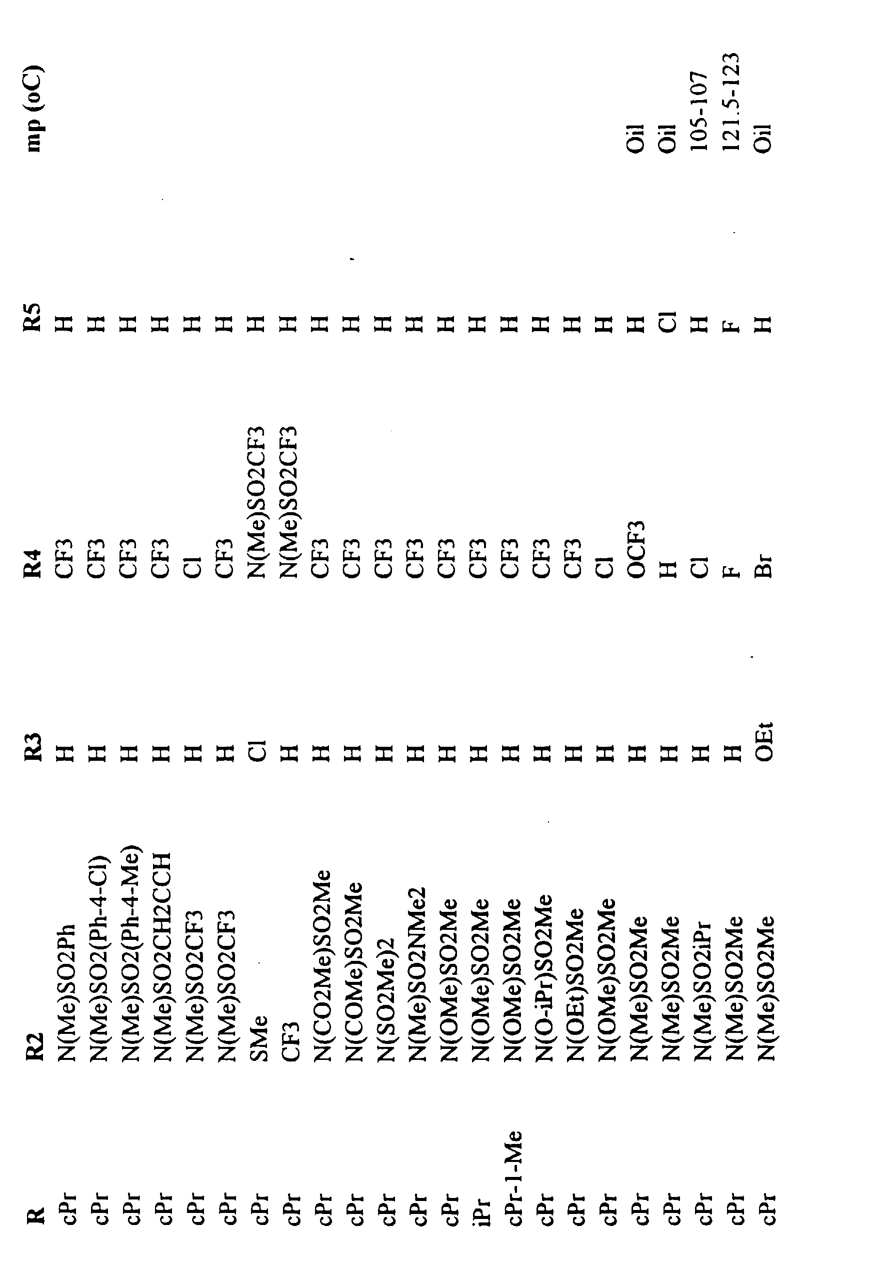

Note: cPr = Cyclopropyl. Also in the Table above, subscripts have not been used, but are understood.

Compounds of formula (I) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders, and that suitable protecting groups may be required to achieve the compounds sought.

According to a feature of the present invention compounds of formula (I) may be prepared from a compound of formula (II): 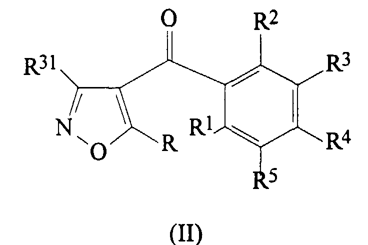 wherein R, R¹, R², R³, R⁴ and R⁵ are as hereinbefore defined and R³¹ represents the hydrogen atom or a group selected from a carboxylic ester, amide, nitrile and acyl.

Where R³¹ represents hydrogen or an acyl group the reaction is carried out by treatment with a base. Examples of suitable bases include alkali or alkaline earth metal hydroxides or alkoxides such as sodium ethoxide or organic bases such as triethylamine.

Where R³¹ represents a group such as an ester, amide or nitrile the conversion is carried out by a hydrolytic reaction. The hydrolytic reaction may be carried out in the presence of an acid or base. Acidic hydrolysis may be achieved for example using aqueous hydrochloric acid. Basic hydrolysis may be achieved for example using sodium hydroxide in a mixture of alcohol and water. The reactions are carried out at a temperature between room temperature and the reflux temperature of the mixture.

According to a further feature of the present invention compounds of formula (I) may be prepared from a compound of formula (III): 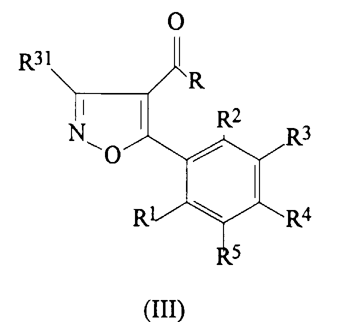 wherein R, R¹, R², R³, R⁴, R⁵ and R³¹ are as hereinbefore defined.

Where R³¹ represents hydrogen or an acyl group the reaction is carried out by treatment with a base. Examples of suitable bases include alkali or alkaline earth metal hydroxides or alkoxides such as sodium ethoxide or organic bases such as triethylamine.

Where R³¹ represents a group such as an ester, amide or nitrile the conversion is carried out by a hydrolytic reaction. The hydrolytic reaction may be carried out in the presence of an acid or base. Acidic hydrolysis may be achieved for example using aqueous hydrochloric acid. Basic hydrolysis may be achieved for example using sodium hydroxide in a mixture of alcohol and water. The reactions are carried out at a temperature between room temperature and the reflux temperature of the mixture.

According to a further feature of the present invention, compounds of formula (I) may also be prepared by the reaction of a benzoyl chloride of formula (IV): 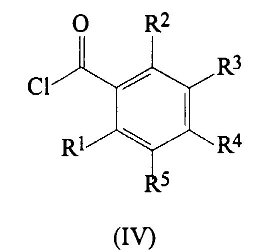 wherein R¹ R², R³, R⁴and R⁵ are as hereinbefore defined, with a beta-ketonitrile of formula(V): 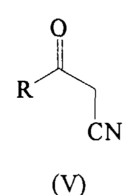 wherein R is as hereinbefore defined. The reaction is generally performed in the presence of a base in a solvent or solvent mixture. Suitable bases include metal hydrides, hydroxides or alkoxides (e.g. sodium or lithium hydride, sodium hydroxide, potassium hydroxide, magnesium ethoxide or magnesium methoxide). Suitable solvents include for example tetrahydrofuran; hydrocarbons such as toluene; or halogenated hydrocarbons such as dichloromethane. The reaction is generally performed at a temperature from 0°C to reflux temperature.

According to a further feature of the present invention, compounds of formula (I) may also be prepared by the reaction of an acid chloride of formula (VI): 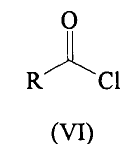 wherein R is as hereinbefore defined, with a beta-ketonitrile of formula (VII): 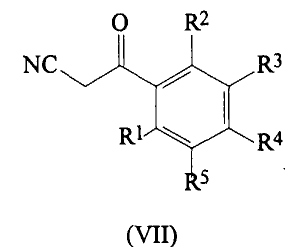 wherein R¹, R², R³, R⁴ and R⁵ are as hereinbefore defined. The reaction is generally performed in the presence of a base in a solvent or solvent mixture. Suitable bases include metal hydrides, hydroxides or alkoxides (e.g. sodium or lithium hydride, sodium hydroxide, potassium hydroxide, magnesium ethoxide or magnesium methoxide). Suitable solvents include for example tetrahydrofuran; hydrocarbons such as toluene; or halogenated hydrocarbons such as dichloromethane. The reaction is generally performed at a temperature from 0°C to reflux temperature.

According to a further feature of the present invention compounds of formula (I) may be prepared by the reaction of a benzoyl chloride of formula (IV) wherein R¹, R², R³, R⁴ and R⁵ are as hereinbefore defined, with a beta-ketonitrile of formula (V) wherein R is as hereinbefore defined, via an intermediate of formula (VIII): 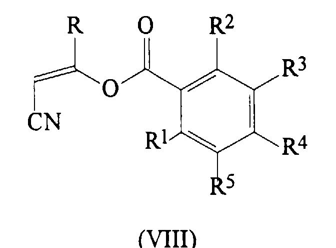 wherein R, R¹, R², R³, R⁴ and R⁵ are as hereinbefore defined. The formation of the intermediate of formula (VIII) may be carried out in the presence of a mild base such as an organic base e.g. triethylamine, in an inert solvent such as acetonitrile or dichloromethane at a temperature between room temperature and the reflux temperature of the mixture. The rearrangement of the intermediate of formula (VIII) to a compound of formula (I) may be carried out optionally in situ in an inert solvent such as acetonitrile or dichloromethane in the presence of a catalyst such as a source of cyanide. Examples of such sources of cyanide are acetone cyanhydrin or an alkali metal cyanide such as potassium cyanide, optionally in the presence of a crown ether such as 18-crown-6.

According to a further feature of the present invention compounds of formula (I) may be prepared by the reaction of an acid chloride of formula (VI) wherein R is as hereinbefore defined, with a beta-ketonitrile of formula (VII) wherein R¹, R², R³, R⁴ and R⁵ are as hereinbefore defined, via an intermediate of formula (IX): 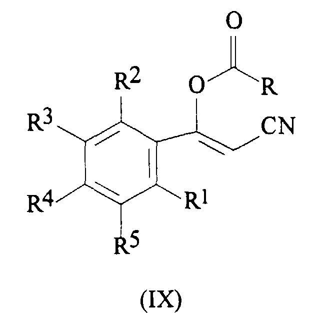 wherein R, R¹, R², R³, R⁴ and R⁵ are as hereinbefore defined. The formation of the intermediate of formula (IX) may be carried out in the presence of a mild base such as an organic base e.g. triethylamine, in an inert solvent such as acetonitrile or dichloromethane at a temperature between room temperature and the reflux temperature of the mixture. The rearrangement of the intermediate of formula (IX) to a compound of formula (I) may be carried out optionally in situ in an inert solvent such as acetonitrile or dichloromethane in the presence of a catalyst such as a source of cyanide. Examples of such sources of cyanide are acetone cyanhydrin or an alkali metal cyanide such as potassium cyanide, optionally in the presence of a crown ether such as 18-crown-6.

Intermediates in the preparation of compounds of formula (I) may be prepared by the application or adaptation of known methods.

Compounds of formula (II) or (III) in which R³¹ represents hydrogen may be prepared by the reaction of a compound of formula (X): 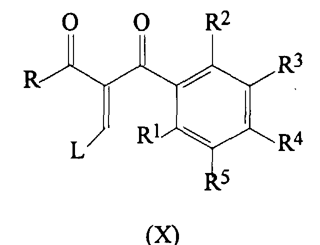 wherein R¹, R², R³, R⁴ and R⁵ are as hereinbefore defined and L is -OR⁷² or -N(R⁷²)₂ and R⁷² is an alkyl group, with a salt of hydroxylamine in the presence of a base or acid acceptor. The reaction is generally carried out using hydroxylamine hydrochloride in the presence of sodium acetate or an organic base such as triethylamine. The reaction is preferably performed in a solvent. Suitable solvents include alcohols such as ethanol or inert solvents such as acetonitrile. The reaction is carried out at a temperature between room temperature and the boiling point of the solvent.

Compounds of formula (X) in which L represents -OR⁷² may be prepared by the reaction of a diketone of formula (XI): 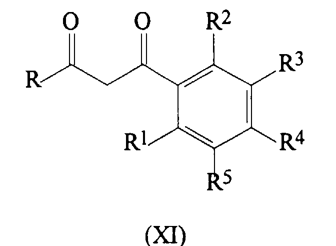 wherein R, R¹, R², R³, R⁴ and R⁵ are as hereinbefore defined, with an ortho ester, HC(OR⁷²)₃. The reaction is generally carried out using triethyl orthoformate in the presence of an acid catalyst such as acetic anhydride. The reaction is carried out at a temperature between room temperature and the boiling point of the mixture.

Compounds of formula (X) in which L represents -N(R⁷²)₂ may be prepared by the reaction of a diketone of formula (XI) with an amide acetal of formula (R⁷²)₂N-CH(OR⁷²)₂. The reaction is optionally carried out in an inert solvent such as toluene at a temperature between room temperature and the boiling point of the mixture.

Compounds of formula (II) wherein R³¹ represents an ester, nitrile or acyl group may be prepared by the reaction of a compound of formula (XII): 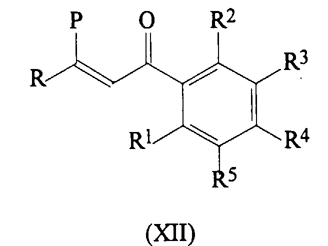 wherein R, R¹, R^{2,} R³, R⁴ and R⁵ are as hereinbefore defined and P is a leaving group such as N,N-dialkylamino, with a compound of formula R³¹-C(Z)=NOH wherein R³¹ represents an ester, nitrile or acyl group and Z is a halogen atom. Generally Z is chlorine or bromine atom. The reaction is generally performed in an inert solvent such as toluene or dichloromethane either in the presence of a base such as triethylamine or a catalyst such as a 4 Angstrom molecular sieve or fluoride ion.

Compounds of formula (XII) may be prepared by the reaction of a compound of formula CH₂=C(R³¹)(P), wherein R³¹ and P are as hereinbefore defined, with a benzoyl chloride of formula (IV). The reaction is generally carried out in the presence of an organic base such as triethylamine in an inert solvent such as toluene or dichloromethane at a temperature between -20°C and room temperature.

Compounds of formula (II) or (III) wherein R³¹ represents an ester, nitrile or acyl group may be prepared by the reaction of a compound of formula (XI) with a compound of formula R³¹-C(Z)=NOH wherein R³¹ represents an ester, nitrile or acyl group and Z is as hereinbefore defined. Generally Z is a chlorine or bromine atom. The reaction is generally performed in an inert solvent such as dichloromethane or acetonitrile in the presence of a base. Examples of suitable bases are alkaline earth metal alkoxides such as magnesium methoxide and the reaction is carried out a temperature between room temperature and the reflux temperature of the mixture.

Compounds of formula (II) or (III) wherein R³¹ represents an amide group may be prepared by the reaction of the corresponding compound of formula (II) or (III) in which R³¹ represents an ester group with ammonia or an amine. The reaction is carried out in a solvent or solvent mixture such as aqueous ethanol at a temperature between room temperature and the reflux temperature of the mixture.

Compounds of formula (III) in which R³¹ represents hydrogen may be prepared by the reaction of a compound of formula (XIII): 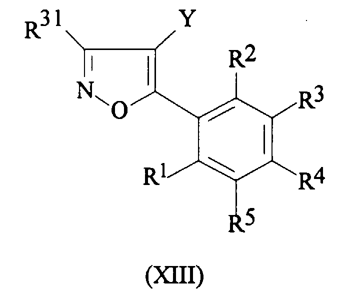 in which R³¹ represents hydrogen and Y represents a carboxy group, or a reactive derivative thereof (such as a carboxylic acid chloride or carboxylic ester) or a cyano group, with an appropriate organometallic reagent such as a Grignard reagent or an organolithium reagent, to introduce the group -COR into the 4-position of the isoxazole ring. The reaction is generally carried out in an inert solvent such as ether or tetrahydrofuran, at a temperature from 0°C to the reflux temperature of the solvent.

Compounds of formula (III) in which R³¹ is an ester, nitrile or acyl group may be prepared by the reaction of a compound of formula (XIV): 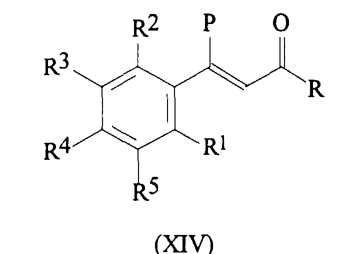 wherein P is a leaving group such as N,N-dialkylamine with a compound of formula R³¹C(Z)=N-OH wherein Z is as hereinbefore defined and R³¹ is an ester, nitrile or acyl group. Generally Z is chlorine or bromine. The reaction is generally performed in an inert solvent such as toluene or dichloromethane either in the presence of a base such as triethylamine or a catalyst such as a 4A molecular sieve or fluoride ion.

Compounds of formula (XIII) in which R³¹ is a hydrogen atom and Y is -CO₂-alkyl or -CN may be prepared by the reaction of a compound of formula (XV): 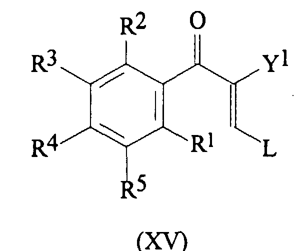 wherein Y¹ represents CO₂-alkyl or -CN and L is as hereinbefore described, with a salt of hydroxylamine such as hydroxylame hydrochloride, in a solvent such as ethanol or acetonitrile, optionally in the presence of a base or acid acceptor such as triethylamine or sodium acetate.

Compounds of formula (XIII) in which R³¹ represents hydrogen and Y represents a carboxylic acid or carboxylic acid chloride may be prepared from the corresponding compound of formula (XIII) in which R³¹ represents hydrogen and Y represents a carboxylic ester group by the hydrolysis of said ester group and conversion, as necessary, of the acid thus obtained to the acid chloride, e.g. by heating with thionyl chloride.

Compounds of formula (XV) may be prepared by the reaction of a compound of formula (VII) or a ketoester of formula (XVI): 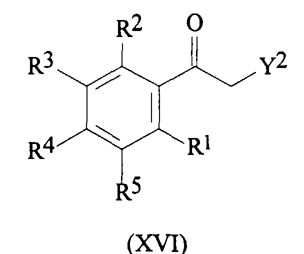 wherein R¹, R², R^{3,} R⁴ and R⁵ are as hereinbefore defined and Y² represents -CO₂-alkyl, with either triethyl orthoformate in the presence of acetic anhydride at the reflux temperature of the mixture or with dimethylformamide dimethylacetal optionally in an inert solvent such as toluene at a temperature from room temperature to the reflux temperature of the mixture.

Compounds of formula (XIV) may be prepared by the reaction of a compound of formula (XVII): 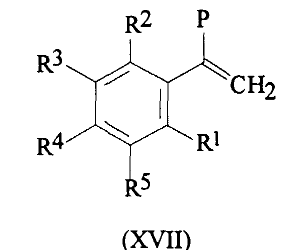 wherein R¹, R², R^{3,} R⁴, R⁵ and P is as hereinbefore defined, with an acid chloride of formula (VI) wherein R is as hereinbefore defined, in an inert solvent such as dichloromethane or toluene, in the presence of a base such as triethylamine.

Acid chlorides of formula (IV) or (VI) are generally known or can be prepared from the corresponding carboxylic acid according to commonly accepted methods, for example by using thionyl chloride in chloroform at reflux.

Beta-ketonitriles of formula (V) may be prepared from acid chlorides of formula (VI) by a number of methods well known in the chemical literature. For example, see Krauss, et al, Synthesis, **1983**, 308, or Muth, et al, J. Org. Chem, **1960**, 25, 736. Alternatively beta-ketonitriles of formula (V) may be prepared by the reaction of an ester of formula R-CO₂Et, wherein R is as hereinbefore defined, with acetonitrile. This reaction is described in the literature for example see the article by Abramovitch and Hauser, J. Am. Chem. Soc., **1942,** 64, 2720.

Beta-ketonitriles of formula (VII) may be prepared from benzoyl chlorides of formula (IV) or from ethyl benzoates of formula (XVIII): 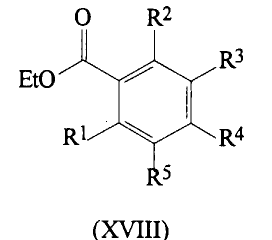 wherein R¹, R², R³, R⁴ and R⁵ are as hereinbefore defined, in a manner analogous to the preparation of beta-ketonitriles of formula (V) set forth above.

Compounds of formula (V), (XI), (XIV), (XVI), (XVII) and (XVIII) are known or may be prepared by the application and adaptation of known methods.

Agriculturally acceptable salts and metal complexes of compounds of formula (I) may be prepared by known methods.

The following examples illustrate the preparation of compounds of general formula (I). In the present specification b.p. means boiling point, m.p. means melting point. Where the letters NMR appear, the characteristics of the proton nuclear magnetic resonance spectrum follow. In the following description cPr represents cyclopropyl; CHex represents cyclohexyl. Unless otherwise specified the percentages are by weight.

It is understood that for preparing the compounds of the invention listed below, certain examples and reference examples are included only for reasons of illustration of the methods employed.

### EXAMPLE 1

Sodium metal (0.11g) was dissolved in absolute ethanol with stirring and was cooled to ambient temperature. 4-(2-Cyclohexylsulphonyl-4-trifluoromethybenzoyl)-5-cyclopropylisoxazole (1.5g) in absolute ethanol was added and the resulting solution was stirred at ambient temperature for 3 hours. It was poured into ice/water, acidified to pH1 with concentrated hydrochloric acid and the resultant white suspension was allowed to warm to ambient temperature before it was extracted with ethyl acetate. The combined organic extracts were washed with water and dried (magnesium sulphate). Evaporation of the solvent gave a light-brown solid (1.3g) of 2-cyano-1-(2-cyclohexylsulphonyl-4-trifluoromethylphenyl)-3-cyclopropylpropan-1,3-dione m.p. 60-62°C.

By proceeding in a similar manner the following compounds of formula (I) were prepared from the appropriately substituted starting materials.

| **R** | **R**^{**1**} | **R**^{**2**} | **R**^{**3**} | **R**^{**4**} | **R**^{**5**} | **mp(**^{**o**}**C) or NMR** |
|---|---|---|---|---|---|---|
| cPr | H | N(Me)SO₂Me | H | NO₂ | H | 203-205 |
| cPr | H | Cl | H | N(Me)SO₂Me | H | 115.8-116.6 |
| cPr | H | NO₂ | H | N(Me)SO₂Me | H | 133-134 |

### EXAMPLE 2

4-(2-Methylsulphonylmethyl-4-bromobenzoyl)-5-cyclopropylisoxazole (0.3g) in dichloromethane was treated at ambient temperature with triethylamine (0.124 ml) and the reaction mixture was stirred at ambient temperature for 3.33 hours. It was treated with 2M hydrochloric acid, diluted with dichloromethane and brine, extracted with more dichloromethane and dried (sodium sulphate). Evaporation of solvent gave a beige gum (0.3 g). This was triturated with diisopropyl ether to give 2-cyano-3-cyclopropyl-1-(2-methylsulphonylmethyl-4-bromophenyl)-propan-1,3-dione, as a white solid (0.24g), m.p. 108°C.

By proceeding in a similar manner the following compounds of formula (I) were prepared from the appropriately substituted starting materials.

| **R** | **R**^{**1**} | **R**^{**2**} | **R**^{**3**} | **R**^{**4**} | **R**^{**5**} | **m.p. (**^{**o**}**C) /NMR** |
|---|---|---|---|---|---|---|
| cPr | H | N(Me)SO₂Me | H | Cl | H | 127.8-128.5 |
| cPr | H | N(Me)SO₂Me | H | Me | H | 106-108 |
| cPr | H | Cl | H | NHSO₂Me | H | 146-152 |
| cPr | H | N(Et)SO₂Me | H | Cl | H | 120-122 |
| cPr | H | N(Me)SO₂Me | H | OCF₃ | H | (a) |
| cPr | H | N(Me)SO₂Me | H | H | Cl | (b) |
| cPr | H | N(Me)SO₂Prⁱ | H | Cl | H | 105-107 |
| cPr | H | N(Me)SO₂Me | H | F | F | 121.5-123 |
| (a) NMR (CDCl₃) 1.2(m,2H), 1.35(m,2H), 2.3(m,1H), 2.8(s,3H), 3.4(s,3H), 7.2(m,2H), 7.6(d,1H), 17.3(bs,1H). (b) NMR (CDCl₃) 1.2(m,2H), 1.35(m,2H), 2.25(m,1H), 2.7(s,3H), 3.3(s,3H), 7.35(d,1H), 7.5(m,2H), 17.2(bs,1H). | | | | | | |

### REFERENCE EXAMPLE 1

A mixture of 3 -cyclopropyl-2-ethoxymethylene-1-(2-phenylsulphenyl-4-trifluoromethylphenyl)propan-1,3-dione (7g), hydroxylarnine hydrochloride (1.24g) and sodium acetate (1.5g) in ethanol was stirred at 25°C for 2 hours. The mixture was then poured into water and extracted with ethyl acetate. The solution was dried over anhydrous sodium sulphate and filtered. The filtrate was evaporated and the residue purified by column chromatography on silica, using a mixture of ethyl acetate and hexane as eluent. The resulting solution was evaporated and the residue crystallised from cyclohexane to give 3.06g of 5-cyclopropyl-4-(2-phenylsulphenyl-4-trifluoromethylbenzoyl)isoxazole, m.p. 115°C.

By proceeding in a similar manner the following compounds of formula (II) were prepared from the appropriately substituted starting materials.

| **R**^{**31**} | **R** | **R**^{**1**} | **R**^{**2**} | **R**^{**3**} | **R**^{**4**} | **R**^{**5**} | **m.p.(**^{**o**}**C) or NMR** |
|---|---|---|---|---|---|---|---|
| H | cPr | H | Cl | H | NHSO₂Me | H | 122.8-124.5 |
| H | cPr | H | N(Me)SO₂Me | H | Cl | H | 128.3-130.8 |
| H | cPr | H | N(Me)SO₂Me | H | OCF₃ | H | a |
| H | cPr | H | N(Me)SO₂Me | H | H | Cl | 111-113 |
| a NMR(CDCl₃) 1.15(m,2H), 1.25(m,2H), 2.6(m,1H), 2.9(s,3H), 3.2(s,3H), 7.2(m,2H), 7.5(d,1H), 8.1(s,1H). | | | | | | | |

### REFERENCE EXAMPLE 2

Hydroxylamine hydrochloride (0.76g) was added to a mixture of 1-[4-chloro-2-(N-ethyl-N-methylsulphonylamino)phenyl]-3-cyclopropyl-2-dimethylaminomethylenepropan-1,3-dione(3.83 g) in ethanol. The mixture was stirred for 1 hour and evaporated to dryness. The residue was dissolved in dichloromethane and washed with water, dried (MgSO₄) and filtered. The filtrate was evaporated to dryness and the residue was purified by chromatography eluted with a mixture of cyclohexane, dichloromethane and ethyl acetate. The product was triturated with a mixture of ether and hexane and filtered to give 4-[4-chloro-2-(N-ethyl-N-methylsulphonylamino)benzoyl]-5-cyclopropylisoxazole (0.81g) as a white solid, m.p. 114-115.8°C.

By proceeding in a similar manner the following compounds of formula (II) above were prepared from the appropriately substituted starting materials.

| **R**^{**31**} | **R** | **R**^{**1**} | **R**^{**2**} | **R**^{**3**} | **R**^{**4**} | **R**^{**5**} | **mp(°C)** |
|---|---|---|---|---|---|---|---|
| H | cPr | H | N(Me)SO₂Me | H | NO₂ | H | 113-115 |
| H | cPr | H | Cl | H | N(Me)SO₂Me | H | 92.4-94.2 |
| H | cPr | H | NO₂ | H | N(Me)SO₂Me | H | 116-117 |
| H | cPr | H | N(Me)SO₂Me | H | Me | H | 120-122 |
| H | cPr | H | N(Me)SO₂Pr¹ | H | Cl | H | 130-132 |
| H | cPr | H | N(Me)SO₂Me | H | F | F | 140-143 |

### REFERENCE EXAMPLE 3

A mixture of 3-cyclopropyl-1-(2-phenylsulphenyl-4-trifluoromethylphenyl)propan-1,3-dione (6.0g) and triethylorthoformate (4.9g) in acetic anhydride was stirred and heated at reflux for 3 hours. It was cooled and evaporated. The residue was treated with toluene and re-evaporated to dryness to give 1-cyclopropyl-2-ethoxymethylene-3-(2-phenylsulphenyl-4-trifluoromethylphenyl)propan-1,3-dione (6.7g) as a red oil.

By proceeding in a similar manner the following compounds of the formula (X) above in which L represents ethoxy were prepared from the appropriately substituted starting materials.

| **R** | **R**^{**1**} | **R**^{**2**} | **R**^{**3**} | **R**^{**4**} | **R**^{**5**} |
|---|---|---|---|---|---|
| cPr | H | Cl | H | NHSO₂Me | H |
| cPr | H | N(Me)SO₂Me | H | Cl | H |
| cPr | H | N(Me)SO₂Me | H | OCF₃ | H |
| cPr | H | N(Me)SO₂Me | H | H | Cl |

### REFERENCE EXAMPLE 4

A mixture of 1-[4-chloro-2-(N-ethyl-N-methyl-sulphonylamino)-phenyl]-3-cyclopropylpropan-1,3-dione (3.5g) and dimethylformamide dimethyl acetal (1.5ml) in dichloromethane was stirred at room temperature overnight and heated at reflux for 3 days. After cooling the mixture was evaporated to dryness to give 1-[4-chloro-2-(N-ethyl-N-methylsulphonylaminophenyl]-3-cyclopropyl-2-dimethylaminomethylenepropan-1,3-dione (3.83g) as an orange gum.

By proceeding in a similar manner the following compounds of the formula (X) above in which L represents N,N-dimethlyamino were prepared from the appropriately substituted starting materials.

| **R** | **R**^{**1**} | **R**^{**2**} | **R**^{**3**} | **R**^{**4**} | **R**^{**5**} |
|---|---|---|---|---|---|
| cPr | H | N(Me)SO₂Me | H | NO₂ | H |
| cPr | H | Cl | H | N(Me)SO₂Me | H |
| cPr | H | NO₂ | H | N(Me)SO₂Me | H |
| cPr | H | N(Me)SO₂Me | H | Me | H |
| cPr | H | N(Me)SO₂Prⁱ | H | Cl | H* |
| cPr | H | N(Me)SO₂Me | H | F | F* |

| | | | | | |
|---|---|---|---|---|---|
| * Reaction performed in toluene | | | | | |

### REFERENCE EXAMPLE 5

A suspension of magnesium (0.47g) in methanol was warmed gently to initiate reaction and then heated at reflux until all of the magnesium had dissolved. It was cooled slightly and t-butyl 3-cyclopropyl-3-oxopropionate (2.39g) was added. The mixture was stirred and heated at reflux for 25 minutes then evaporated to dryness. It was dissolved in toluene and re-evaporated to dryness. The residue was again dissolved in toluene and 2-chloro-4-(methylsulphonylamino)benzoyl chloride (3.2g) was added. The mixture was stirred at room temperature overnight. Hydrochloric acid (2M) was added and the mixture was stirred. The layers were separated and the organic layer was dried (MgSO₄) and filtered. The filtrate was evaporated to give t-butyl 2-[2-chloro-4-(methylsulphonylamino)benzoyl]-3-cyclopropyl-3-oxopropionate (3.7g) as a white solid, m.p. 137-140°C.

t-Butyl 2-[2-chloro-4-(methylsulphonylamino)benzoyl]-3-cyclopropyl-3-oxopropionate (2g) was dissolved in toluene and p-toluenesulphonic acid (0.2g) was added. The mixture was stirred and heated at reflux for 0.5 hours. It was cooled, washed with water, dried (MgSO₄) and filtered. The filtrate was evaporated to dryness to give 1-[2-chloro-4-(methylsulphonylamino)phenyl]-3-cyclopropylpropan-1,3-dione (1.48g) as a white solid, m.p. 119.9-121.6°C.

By proceeding in a similar manner the following compounds of formula (XI) above were prepared from the appropriately substituted starting materials without isolation of the intermediate ester.

| **R** | **R**^{**1**} | **R**^{**2**} | **R**^{**3**} | **R**^{**4**} | **R**^{**5**} | **m.p. (**^{**o**}**C) or NMR** |
|---|---|---|---|---|---|---|
| cPr | H | N(Me)SO₂Me | H | Cl | H | 93.3-96.5 |
| cPr | H | N(Me)SO₂Me | H | NO₂ | H | - |
| cPr | H | N(Et)SO₂Me | H | Cl | H | a |
| cPr | H | Cl | H | N(Me)SO₂Me | H | - |
| cPr | H | NO₂ | H | N(Me)SO₂Me | H | - |
| cPr | H | N(Me)SO₂Me | H | Me | H | b |
| cPr | H | N(Me)SO₂Me | H | OCF₃ | H | c |
| cPr | H | N(Me)SO₂Me | H | H | Cl | 110-113 |
| cPr | H | N(Me)SO₂Prⁱ | H | Cl | H | d (note 1) |
| cPr | H | N(Me)SO₂Me | H | F | F | 104-105 (note1) |
| Note 1 - The trione intermediate was stirred overnight in trifluoroacetic acid a NMR(CDCl₃) 0.9-1.4(m,7H) 1.7-1.9(m,1H) 3.0(s,3H) 3.65(q,2H) 6.1(s,1H) 7.4(m,2H) 7.55(d,1H) 16.0-16.3(bs,1H) b NMR(CDCl₃) 0.9-1.1(m,2H) 1.15-1.25(m,2H) 1.7-1.85(m,1H) 2.35(s,3H) 2.95(s,3H) 3.25(s,3H) 6.1(s,1H) 7.15(d,1H) 7.2(s,1H) 7.5(d,1H) 16.1-16.3(bs,1H). c NMR(CDCl₃) 1.0(m,2H), 1.2(m,1H), 1.7(m,1H), 3.0(s,3H), 3.25(s,3H), 6.05(s,1H), 7.25(m,2H), 7.6(d,1H), 16.0(s,1H). d NMR(CDCl₃) 1.0-1.4(m,4H), 1.45(d,6H), 1.8(m,1H), 3.35(s,3H), 3.3(m,1H), 6.1(s,1H), 7.35(m,3H), 16.15(bs,1H). | | | | | | |

Benzoyl chlorides were prepared by heating the appropriately substituted benzoic acids with thionyl chloride. The excess thionyl chloride was removed by evaporation the benzoyl chlorides thus obtained were used without further purification.

### REFERENCE EXAMPLE 6

A mixture of 2-phenylsulphenyl-4-trifluoromethylbenzoic acid (11.5g), thionyl chloride (11.4g), dimethylformamide (0.2ml) and dichloroethane was heated at reflux for 90 minutes. The solution was then concentrated under reduced pressure and the residue was dissolved in methanol and heated at reflux for one hour. The resulting solution was poured into aqueous sodium bicarbonate and extracted with ether. The organic phase was dried over anhydrous sodium sulphate, filtered and evaporated. The resulting material was crystallised from hexane to give methyl 2-phenylsulphenyl-4-trifluoromethylbenzoate (10.5g) as white crystals, m.p. 58^{o}C.

By proceeding in a similar manner the following compounds were prepared:
4-chloro-2-(N-methyl-N-isopropylsulphonylamino)benzoyl chloride. For this preparation neat thionyl chloride was employed.
4,5-difluoro-2-(N-methyl-N-methylsulphonylamino)benzoyl chloride, using neat thionyl chloride.

### REFERENCE EXAMPLE 7

A mixture of 2-phenylsulphenyl-4-trifluoromethylbenzonitrile (9g), concentrated sulphuric acid (27 ml) and water was heated at reflux for 10 hours. The mixture was then cooled, poured into water and extracted with dichloromethane. The organic extract was extracted with aqueous sodium hydroxide. The resulting aqueous solution was acidified with aqueous hydrochloric acid to pH 1. The suspension was extracted with dichloromethane, dried over anhydrous sodium sulphate, filtered and after evaporation of the dichloromethane 2-phenylsulphenyl-4-trifluoromethylbenzoic acid was obtained as a white solid (7.5g), m.p. 161°C.

### REFERENCE EXAMPLE 8

2N Sodium hydroxide solution (20 ml) was added to a stirred solution of methyl 4-chloro-2-(N-methyl-N-methylsulphonylamino)benzoate (2.75 g) in methanol. The mixture was stirred at reflux for 0.5 hours. Alter cooling, the mixture was acidified with 2N hydrochloric acid and extracted with ethyl acetate. The organic extracts were combined, dried and evaporated to yield 4-chloro-2-(N-methyl-N-methylsulphonylamino)benzoic acid as a white solid (2.45g), m.p. 161-164^{o}C.

By proceeding in a similar manner, the following compounds were prepared:-
2-(N-methyl-N-methylsulphonylamino)-4-nitrobenzoic acid NMR (DMSO-d₆) 3.1(s,3H) 3.3(s,3H) 7.95(d,1H) 8.3(d,1H) 8.4(s,1H) 13.3-13.8(bs,1H);
4-chloro-2-(N-ethyl-N-methylsulphonylamino)benzoic acid m.p. 148-151°C;
2-chloro-4-(N-methyl-N-methylsulphonylamino)benzoic acid m.p.152-153^{o}C;
4-(N-methyl-N-methylsulphonylamino)-2-nitrobenzoic acid m.p. 177-178.6^{o}C;
4-methyl-2-(N-methyl-N-methylsulphonylamino)benzoic acid m.p. 185-187^{o}C;
2-(N-methyl-N-methylsulphonylamino)-4-trifluoromethoxybenzoic acid, m.p. 139-142.5°C;
5-chloro-2-(N-methyl-N-methylsulphonylamino)benzoic acid, NMR(D₆-DMSO) 3.0(s,3H), 3.2(s,3H), 7.6(d,1H), 7.65(m,1H), 7.7(m,1H);

### REFERENCE EXAMPLE 9

Potassium carbonate (12.5g) was added to a stirred solution of methyl 4-chloro-2-(N-methylsulphonylamino)benzoate (7.5g) in acetone. The mixture was stirred for 15 minutes and methyl iodide (8.0g) was added. The resultant mixture was stirred at room temperature for 1 hour and left to stand overnight. The mixture was evaporated to dryness and the residue was dissolved in ethyl acetate and washed with sodium hydroxide solution (2M) and water, dried (anhydrous MgSO₄) and filtered. The filtrate was evaporated to dryness to give methyl 4-chloro-2-(N-methyl-N-methylsulphonylamino)benzoate (4.9g) as a white solid, m.p. 73-75^{o}C.

By proceeding in a similar manner the following compounds were prepared from the appropriately substituted starting materials.
methyl 2-chloro-4-(N-methyl-N-methylsulphonylamino)benzoate, NMR (DMSO-d₆) 3.05(s,3H) 3.35(s,3H) 3.85(s,3H) 7.5(d,1H) 7.6(s,1H) 7.85(d,1H);
methyl 2-(N-methyl-N-methylsulphonylamino)-4-nitrobenzoate, NMR (DMSO-d₆) 3.05(s,3H) 3.25(s,3H) 3.8(s,3H) 8.0(d,1H) 8.3(d,1H) 8.4(s,1H);
ethyl 4-chloro-2-(N-ethyl-N-methylsulphonylamino)benzoate, NMR(CDCl₃) 1.1(t,3H) 1.35(t,3H) 2.9(s,3H) 3.65(q,2H) 4.3(q,2H) 7.3(d,1H) 7.35(s,1H) 7.8(d,1H);
methyl 2-(N-methyl-N-methylsulphonylamino)-4-trifluoromethoxybenzoate, NMR(CDCl₃) 2.95(s,3H), 3.3(s,3H), 3.95(s,3H), 7.25(m,2H), 8.0(d,1H);
methyl 5-chloro-2-(N-methyl-N-methylsulphonylamino)benzoate, m.p. 91-93°C.

### REFERENCE EXAMPLE 10

Methyl iodide (22.0 ml) was added to a stirred suspension of 4-methyl-2-(N-methylsulphonylamino)benzoic acid (8.0g) and anhydrous potassium carbonate (24.2g) in acetone and the mixture was stirred and heated at reflux overnight. The mixture was cooled and filtered and the filtrate was evaporated to dryness. The residue was dissolved in dichloromethane and washed with aqueous sodium bicarbonate solution, water, dried (MgSO₄) and filtered. The filtrate was evaporated to dryness to give methyl 4-methyl-2-(N-methyl-N-methylsulphonylamino)benzoate (8.36g) as a cream solid, m.p. 100-103°C.

By proceeding in a similar manner methyl 4-(N-methyl-N-methylsulphonylamino)-2-nitrobenzoate, NMR (CDCl₃) 2.95(s,3H) 3.4(s,3H) 3.9(s,3H) 7.7(d,1H) 7.75(d,1H) 7.85(s,1H) was prepared from the appropriately substituted starting material.

### REFERENCE EXAMPLE 11

A solution of methanesulphonyl chloride (6.3g) in dichloromethane was added to a stirred, cooled (0-5°C) solution of methyl 2-amino-4-chlorobenzoate (9.5g) in dichloromethane. Triethylamine (7.1g) was then added and the mixture was stirred at 0-5°C for 10 minutes and then at room temperature for 0.5 hours.

The mixture was diluted with 2N hydrochloric acid. The organic phase was separated, washed with water, dried and evaporated.

The crude product was purified by column chromatography to yield methyl 4-chloro-2-(N-methylsulphonylamino)benzoate as a white solid, (3.6g) m.p. 125.5-128.1°C.

### REFERENCE EXAMPLE 12

Concentrated sulphuric acid (20 ml) was added to a suspension of 2-chloro-4-(N-methylsulphonylamino)benzoic acid (10.3g) in methanol and the mixture was stirred and heated at reflux for 22 hours. It was cooled, evaporated to dryness and diluted with water, extracted with ethyl acetate, washed with aqueous sodium bicarbonate solution, water, dried (MgSO₄) and filtered. The filtrate was evaporated to dryness to give methyl 2-chloro-4-(N-methylsulphonylamino)benzoate(10.0g) as a off-white solid, NMR (DMSO-d₆) 3.15(s,3H) 3.85(s,3H) 7.2(d,1H) 7.3(s,1H) 7.85(d,1H) 10.5(s,1H).

By proceeding in a similar manner the following compounds were prepared:
methyl 2-(N-methylsulphonylamino)-4-nitrobenzoic acid NMR (CDCl₃) 3.15 (s,3H) 4.0(s,3H) 7.9(d,1H) 8.25(d,1H) 8.5(s,1H) 10.65(s,1H);
methyl 3,4-difluoro-2-methylbenzoate, m.p. 44-45.8°C.

### REFERENCE EXAMPLE 13

A mixture of 2-chloro-4-(N-methylsulphonylamino)benzoic acid and 2-chloro-4-[N,N-bis(methysulphonyl)amine]benzoic acid (3.6g) in aqueous sodium hydroxide (2M) and methanol was stirred and heated at reflux for 0.5 hours. It was cooled and the methanol was removed by evaporation. The aqueous residue was acidified and the product was filtered off to give 2-chloro-4-(N-methylsulphonylamino)benzoic acid (3.4g) as a white solid, m.p. 256-258^{o}C.

By proceeding in a similar manner the following compound was prepared from the appropriately substituted starting materials. 2-(N-methylsulphonylamino)-4-nitrobenzoic acid, NMR (DMSO-d₆) 3.3(s,3H) 7.9(d,1H) 8.2(d,1H) 8.35(s,1H) 10.5-11.1(bs,1H).

### REFERENCE EXAMPLE 14

A solution of sodium hydroxide (2.38g) in water was added to a mixture of methyl 2-(N-methylsulphonylamino)-4-trifluoromethoxybenzoate and methyl 2-[N,N-bis(methylsulphonyl)amino]-4-trifluoromethoxybenzoate(8.26g) in methanol at 15-20^{o}C. After an additional 15 minutes the solid was filtered, dissolved in ethyl acetate, dried (magnesium sulphate) and evaporated in vacuo to give methyl 2-(N-methylsulphonylamino)-4-trifluoromethoxybenzoate as a brown solid (4.15g) after trituration with hexane, NMR (CDCl₃) 3.0(s,3H), 3.9(s,3H), 6.9(m,1H), 7.55(m,1H), 8.05(d,1H), 10.5(brs,1H).

### REFERENCE EXAMPLE 15

An aqueous solution of sodium hydroxide (11.0g) was added to a solution of a mixture of methyl 4-methyl-2-(N,N-bis(methysulphonyl)amino]benzoate and methyl 4-methyl-2-(N-methylsulphonylamino)benzoate(23.26g) in methanol and the resulting suspension was heated at reflux for 1 hour. It was cooled and the methanol was removed by evaporation. The aqueous solution was acidified and the resultant solid was filtered off to give 4-methyl-2-(N-methylsulphonylamino)benzoic acid (16.42g) as a cream solid, m.p. 202-205°C.

By proceeding in a similar manner the following compound was prepared from the appropriately substituted starting materials.

4-(N-methylsulphonylamino)-2-nitrobenzoic acid, NMR (DMSO-d₆) 3.2(s,3H) 7.5(d,1H) 7.6(s,1H) 7.9(d,1H) 10.8(s,1H) 13.3-14.1(bs,1H).

### REFERENCE EXAMPLE 16

Methanesulphonyl chloride (5.72g) was added to a stirred, cooled (0°C) mixture of 4-amino-2-chlorobenzoic acid (6.9g) and triethylamine (13.1g) in acetonitrile. The mixture was then stirred at room temperature for 3.5 hours. Triethylamine (4g) was added and the mixture was cooled to 0°C and further methanesulphonyl chloride (3.8g) was added. The mixture was then stirred at room temperature for one hour. The mixture was filtered and the filtrate evaporated. The residue was dissolved in 2N sodium hydroxide solution and washed with diethyl ether. The aqueous solution was acidified to pH 2-3 with 2N hydrochloric acid and then extracted with ethyl acetate. The organic extracts were evaporated. The residue was triturated with diethyl ether to yield a mixture of 2-chloro-4-(N-methylsulphonylamino)benzoic acid and 2-chloro-4-[N,N-bis(methylsulphonyl)amino]benzoic acid.

### REFERENCE EXAMPLE 17

Methanesulphonyl chloride (12.2ml) was added to a stirred, cooled solution of methyl 2-amino-4-methylbenzoate (10.3g) and triethylamine (19.5ml) in dichloromethane while maintaining the temperature below 0°C. The mixture was stirred at room temperature for 4 hours. Hydrochloric acid (2M) was added and the layers were separated. The organic layer was washed with water, dried (MgSO₄) and filtered. The filtrate was evaporated to dryness to give a mixture of methyl 4-methyl-2-[N,N-bis(methylsulphonyl)amino]benzoate and methyl 4-methyl-2-(N-methylsulphonylamino)benzoate (18.26g) as a yellow solid which was not further purified.

By proceeding in a similar manner the following compounds were prepared:
4-[N,N-bis-(methylsulphonyl)amino]-2-nitrobenzoic acid;
methyl 2-[N,N-bis(methylsulphonyl)amino]-4-trifluoromethoxybenzoate and methyl 2-(N-methylsulphonylamino)-4-trifluoromethoxybenzoate;
methyl 5-chloro-2-(N-methylsulphonylamino)benzoate and methyl 5-chloro-2-[N,N-bis(methylsulphonyl)amino]benzoate.

### REFERENCE EXAMPLE 18

Oxalyl chloride (2.3ml) was added to a stirred solution of 2-(N-methyl-N-methylsulphonylamino)-4-trifluoromethoxybenzoic acid (6.2g) in 1,2-dichloroethane, N,N-Dimethylformamide (3 drops) was added and stirring maintained for 2 hours. After evaporation, 2-(N-methyl-N-methylsulphonylamino)-4-trifluoromethoxybenzoyl chloride (6.3g) was obtained as a red oil and used directly in the next stage.

By proceeding in a similar manner the following compounds were prepared:
5-chloro-2-(N-methyl-N-methylsulphonylamino)benzoyl chloride;
4-chloro-2-methylsulphenyl-3 -isopropylsulphenylmethyl benzoyl chloride;

### REFERENCE EXAMPLE 19

A solution of sodium hydroxide (3.56g) in water (25ml) was added dropwise during 15 minutes to a mixture of methyl 5-chloro-2-(N-methylsulphonylamino)benzoate and methyl 5-chloro-2-[N,N-bis(methylsulphonyl)amino]benzoate (9.9g) in methanol maintained at 20°C. After a further 15 minutes, the solvent was concentrated in vacuo and acidified with potassium bisulphate. The precipitated product was filtered, washed (water) and dried to give methyl 5-chloro-2-(N-methylsulphonylamino)benzoate (6.69g), m.p. 117-120^{o}C.

### REFERENCE EXAMPLE 20

N-Methyl isopropylsulphonamide (5.0g) was added dropwise to a stirred mixture of sodium hydride (60% oil dispersion, 2.9g) in dry dioxan (70ml). When the effervescence had subsided 2-bromo-4-chlorobenzoic acid (7.8g) was added portionwise. When the liberation of hydrogen had ceased, copper (I) bromide (0.95g) was added and the mixture heated overnight under reflux conditions. The solvent was evaporated, hydrochloric acid (2m) added and the mixture extracted (ethyl acetate). The extract was dried (magnesium sulphate), evaporated and triturated with ether to give 4-chloro-2-(N-methyl-N-isopropylsulphonylamino)benzoic acid (6.45g), m.p. 178-180^{o}C.

By proceeding in a similar manner the following compound was prepared:
4,5-difluoro-2-(N-methyl-N-methylsulphonylamino)benzoic acid from 2-chloro-4,5-difluorobenzoic acid and employing copper (I) chloride instead of copper (I) bromide.

According to a feature of the present invention, there is provided a method for controlling the growth of weeds (i.e. undesired vegetation) at a locus which comprises applying to the locus a herbicidally effective amount of at least one 2-cyano-1,3-dione derivative of general formula (I) or a agriculturally acceptable salt thereof. For this purpose, the 2-cyano-1,3-dione derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (e.g. grass) weeds by pre- and/or, post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of
* broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and
* grass weeds, for example Alopecurus myosuroides, Avena fatua, Digitaria sanguinalis, Echinochloa crus-galli, Eleusine indica and Setaria spp, e.g. Setaria faberii or Setaria viridis, and
* sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01 kg and 5 kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is a area used, or to be used, for growing of crops, e.g. the crops hereinbefore mentioned, application rates between 0.01 kg and 4.0 kg, and preferably between 0.01 kg and 2 kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates between 0.25 kg and 5 kg, and preferably between 0.5 kg and 4 kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1 kg and 20 kg, and preferably between 5 and 10 kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the 2-cyano- 1,3-dione derivatives of general formula (I) or a agriculturally-acceptable salt thereof in association with, and preferably homogeneously dispersed in, one or more compatible herbicidally-acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is a oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are
* aqueous suspension concentrates which comprise from 10 to 70% of one or more compounds of general formula (I), from 2 to 10% of surface-active agent, from 0.1 to 5% of thickener and from 15 to 87.9% of water,
* wettable powders which comprise from 10 to 90% of one or more compounds of general formula (I), from 2 to 10% of surface-active agent and from 8 to 88% of solid diluent or carrier,
* soluble powders which comprise from 10 to 90% of one or more compounds of general formula (I), from 2 to 40% of sodium carbonate and from 0 to 88% of solid diluent
* liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water,
* liquid emulsifiable suspension concentrates which comprise from 10 to 70% of one or more compounds of general formula (I), from 5 to 15% of surface-active agent, from 0.1 to 5% of thickener and from 10 to 84.9% of organic solvent
* granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and,
* emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1- methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2-dimethyl-3,5-diphenyl-pyrazolium salts], flampropmethyl [methyl N-2-(N-benzoyl-3-chloro-4-fluoroanilino)-propionate], fluometuron [N'-(3-trifluoro- methylphenyl)-N,N-dimethylurea], isoproturon [N'-(4-isopropylphenyl)-N,N-dimethylurea], nicosulfuron [2-(4,6-dimethoxypyrimidin-2-ylcarbamoylsulfamoyl)-N,N-dimethylnicotinamide] insecticides, e.g. synthetic pyrethroids, e.g. permethrin and cypermethrin, and fungicides, e.g. carbamates, e.g. methyl N-(1-butyl-carbamoylbenzimidazol-2-yl)carbamate, and triazoles e.g. 1-(4-chloro-phenoxy)-3,3- dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the 2-cyano-1,3-dione derivatives of general formula (I) or a agriculturally-acceptable salt thereof or, as is preferred, a herbicidal composition as hereinbefore described, and preferably a herbicidal concentrate which must be diluted before use, comprising at least one of the 2-cyano-1,3-dione derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the 2-cyano-1,3-dione derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01 kg and 20 kg of active material per hectare in the manner and for the purposes hereinbefore described.

The following Examples illustrate herbicidal compositions according to the present invention:

### EXAMPLE C1

A wettable powder is formed from:
* active ingredient (compound 1): 50% w/w
* nonylphenol/ethylene oxide condensate containing 9 moles of ethylene oxide per mol of phenol: 5% w/w
* silicon dioxide of micro-fine particle size: 5% w/w
* synthetic magnesium silicate carrier: 40% w/w
by absorbing the condensate on the silicon dioxide, mixing with the other ingredients and grinding the mixture in a hammermill to give a wettable powder.

Similar wettable powders may be prepared as described above by replacing the 2-cyano-1,3-dione (compound 1) by other compounds of formula (I).

### EXAMPLE C2

An aqueous suspension concentrate is formed from:
* active ingredient (compound 1): 50% w/v
* nonylphenol/ethylene oxide condensate containing 9 moles of ethylene oxide per mol of phenol: 1% w/v
* sodium salt of polycarboxylic acid: 0.2% w/v
* Ethylene glycol : 5% w/v
* polysaccaride xanthan gum thickener: 0.15% w/v
* water to 100% by volume
by intimately mixing the ingredients and grinding in a ball-mill for 24 hours.

Similar aqueous concentrates may be prepared as described above by replacing the 2-cyano-1,3-dione (compound 1) by other compounds of general formula (I).

Representative compounds of formula (I) have been used in herbicidal applications according to the following procedures.

### Method of use of herbicidal compounds :

### Herbicidal activity:

Appropriate quantities of the compounds used to treat the plant were dissolved in acetone to give solutions equivalent to an application rate of up to 1000g of the compounds used to treat the plants per hectare (g/ha). These solutions were applied at 260 litres of spray fluid per hectare.

### a) Pre-emergence application weed control.

Seeds (weeds or crops) were sown in loam soil pots.

The compounds of the invention were applied to the soil surface as described above.

### b) Post-emergence application weed control.

Weed species were grown until ready for spraying with the compounds of the invention. The growth stage of the plants at spraying were as follows:

| 1) Broad-leafed weeds : | |
|---|---|
| Abutilon theophrasti | 1-2 leaves. |
| Amaranthus retroflexus | 1-2 leaves. |
| Galium aparine | 1-2 whorls. |
| Sinapis arvensis | 2 leaves. |
| Ipomoea purpurea | 1-2 leaves. |
| Xanthium strumarium | 2 leaves. |

| 2) Grass weeds : | |
|---|---|
| Alopecurus myosuroides | 2 leaves. |
| Avena fatua | 1-2 leaves. |
| Echinochloa crus-galli | 2-3 leaves. |
| Setaria viridis | 2-3 leaves. |

| 3) Sedges : | |
|---|---|
| Cyperus esculentus | 3 leaves. |

### c) Crop tolerance

Compounds of the invention were applied pre-emergence as in (a) or post emergence (3-leaf stage) to the following crops:- wheat, maize, rice, soya and cotton.

A single pot of each plant species was allocated to each treatment with unsprayed controls and controls sprayed with acetone alone.

After treatment, the pots were kept in the greenhouse and were watered overhead.

Visual assessment of phytotoxicity was made 17-20 days after spraying. Weed control results were expressed as the percentage reduction in growth or kill of the weeds, in comparison with the plants in the control pots. Crop tolerance was expressed as the percentage damage to crop.

Representative compounds of the invention, when used at an application rate of 1 kg/ha or less, show herbicidal activity against one or more of the weed species listed above: such compounds also show selectivity on one or more of the listed crop species.

## Claims

1. A 2-cyano-1,3-dione derivative of the formula: 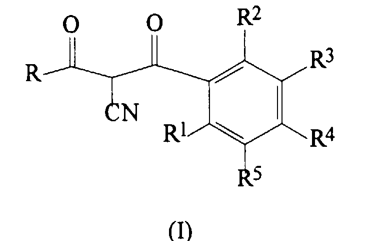 wherein:
R represents:-
a straight- or branched- chain alkyl group containing up to six carbon atoms which is optionally substituted by one or more halogen atoms; or
a cycloalkyl group containing from three to six carbon atoms optionally substituted by one or more R⁷ groups;
R¹ represents the hydrogen atom;
R², R³, R⁴ and R⁵, which may be the same or different, each represents:-
the hydrogen atom;
a straight- or branched- chain alkyl, alkenyl or alkynyl group containing up to six carbon atoms optionally substituted by one or more halogen atoms;
a straight- or branched- chain alkyl group containing up to six carbon atoms which is substituted by a group -OR⁶;
a halogen atom;
phenyl optionally substituted by from one to three groups R²¹ which may be the same or different;
a group selected from -COR⁷, nitro, cyano, -CO₂R⁶, -S(O)ₙR⁹, -O(CH₂)ₘOR⁶, -N(R¹²)SO₂R⁸, -CONR¹⁰R¹⁵ and -OR⁶¹;
provided that at least one of the groups R² to R⁵ represents -N(R¹²)SO₂R⁸;
R⁶ represents:-
a straight- or branched- chain alkyl, alkenyl or alkynyl group containing up to six carbon atoms optionally substituted by one or more halogen atoms; or
a cycloalkyl group containing from three to six carbon atoms;
R⁶¹ represents:-
a straight- or branched- chain alkyl, alkenyl or alkynyl group containing up to six carbon atoms optionally substituted by one or more halogen atoms;
a cycloalkyl group containing from three to six carbon atoms;
or phenyl optionally substituted by from one to five groups R²¹ which may be the same or different;
R⁷ represents:-
a straight- or branched- chain alkyl group containing up to six carbon atoms optionally substituted by one or more halogen atoms;
or a cycloalkyl group containing from three to six carbon atoms;
R⁸ represents:-
a straight- or branched- chain alkyl, alkenyl or alkynyl group containing up to six carbon atoms optionally substituted by one or more halogen atoms;
a cycloalkyl group containing from three to six carbon atoms;
phenyl optionally substituted by from one to five groups R²¹ which may be the same or different; or -NR¹⁰R¹¹;
R⁹ represents:-
a straight- or branched- chain alkyl, alkenyl or alkynyl group containing up to six carbon atoms optionally substituted by one or more halogen atoms;
a cycloalkyl group containing from three to six carbon atoms;
or phenyl optionally substituted by from one to five groups R²¹ which may be the same or different;
R¹⁰ represents hydrogen or a straight- or branched- chain alkyl group containing up to six carbon atoms optionally substituted by one or more halogen atoms;
R¹¹ represents:-
a straight- or branched- chain alkyl group containing up to six carbon atoms optionally substituted by one or more halogen atoms;
where R¹⁰ and R¹¹ are part of a group -NR¹⁰R¹¹ they may, together with the nitrogen to which they are attached, form a five or six membered ring optionally having one additional heteroatom in the ring which is oxygen or nitrogen, wherein the ring is optionally substituted by one or more alkyl groups containing up to three carbon atoms;
R¹² represents:
the hydrogen atom; or
a straight- or branched- chain alkyl, alkenyl or alkynyl group containing up to six carbon atoms optionally substituted by one or more halogen atoms;
a cycloalkyl group containing from three to six carbon atoms;
phenyl optionally substituted by from one to five groups R²¹ which may be the same or different; or
a group -OR¹⁷
R¹⁵ represents a group selected from R⁷ and -OR¹⁷;
where R¹⁰ and R¹⁵ are part of a group -CONR¹⁰R¹⁵ they may, together with the nitrogen to which they are attached, form a 5 or 6 membered ring optionally having one additional heteroatom in the ring which is oxygen or nitrogen, wherein the ring is optionally substituted by one or more alkyl groups containing up to 3 carbon atoms;
R¹⁷ represents a straight- or branched- chain alkyl group containing up to six carbon atoms;
R²¹ represents
a halogen atom;
a straight- or branched- chain alkyl group containing up to three carbon atoms optionally substituted by one or more halogen atoms;
or a group selected from nitro, cyano, -S(O)ₙR⁷ and -OR⁷;
m represents one, two or three; and
n represents zero, one or two
or an agriculturally acceptable salt, metal complex or enolic tautomeric form thereof.

2. A compound according to claim 1 wherein:
R represents:-
a straight- or branched- chain alkyl group containing up to six carbon atoms which is optionally substituted by one or more halogen atoms; or
a cycloalkyl group containing from three to six carbon atoms optionally substituted by one or more methyl groups;
R², R³, R⁴ and R⁵, which may be the same or different, each represents:-
a hydrogen or halogen atom;
a straight- or branched- chain alkyl, alkenyl or alkynyl group containing up to six carbon atoms optionally substituted by one or more halogen atoms;
a straight- or branched- chain alkyl group containing up to six carbon atoms which is substituted by a group -OR⁶;
phenyl optionally substituted by from one to three groups R²¹ which may be the same or different;
or a group selected from -COR⁷, cyano, nitro, -CO₂R⁶, -S(O)ₙR⁹, -O(CH₂)ₘOR⁶, -N(R¹²)SO₂R⁸ and -OR⁶¹;
R⁶ and R⁷ which may be the same or different, each represents:-
a straight- or branched- chain alkyl group containing up to six carbon atoms optionally substituted by one or more halogen atoms;
or a cycloalkyl group containing from three to six carbon atoms;
R⁶¹ and R⁹, which may be the same or different, each represents:-
a straight- or branched- chain alkyl or alkenyl group containing up to six carbon atoms optionally substituted by one or more halogen atoms;
a straight- or branched- chain alkynyl group containing from three to six carbon atoms;
a cycloalkyl group containing three to six carbon atoms;
R⁸ represents:-
a straight- or branched- chain alkyl or alkenyl group containing up to six carbon atoms optionally substituted by one or more halogen atoms;
a straight- or branched- chain alkynyl group containing from three to six carbon atoms;
a cycloalkyl group containing three to six carbon atoms; or
phenyl optionally substituted by from one to three groups R²¹ which may be the same or different;
R¹² represents:-
the hydrogen atom; or
a straight- or branched- chain alkyl or alkenyl group containing up to six carbon atoms optionally substituted by one or more halogen atoms;
a straight- or branched- chain alkynyl group containing from three to six carbon atoms;
or a cycloalkyl group containing three to six carbon atoms;
R²¹ represents:-
a halogen atom;
a straight- or branched- chain alkyl group containing up to three carbon atoms optionally substituted by one or more halogen atoms; or
a group selected from nitro, cyano, -S(O)ₙR⁷ or -OR⁷;
m represents two or three; and
n represents zero, one or two.

3. A compound according to claim 1 wherein:
R represents:-
a straight- or branched- chain alkyl group containing up to three carbon atoms; or
a cycloalkyl group containing three or four carbon atoms optionally substituted by a methyl group;
R², R³ and R⁴ which may be the same or different, each represents:-
a hydrogen, chlorine, bromine or fluorine atom; or
a straight- or branched- chain alkyl, alkenyl or alkynyl group containing up to four carbon atoms optionally substituted by one or more halogen atoms;
a straight- or branched- chain alkyl group containing up to four carbon atoms which is substituted by a group -OR⁶; or
a group selected from -COR⁷, -CO₂R⁶, -S(O)ₙR⁹, -O(CH₂)ₘOR⁶, -N(R¹²)SO₂R⁸ and -OR⁶¹;
R⁵ represents the hydrogen atom;
R⁶, R⁷, and R⁹ which may be the same or different, each represents:-
a straight- or branched- chain all group containing up to four carbon atoms optionally substituted by one or more chlorine, bromine or fluorine atoms; or
a cyclopropyl group;
R⁶¹ and R⁸, which may be the same or different, each represents:-
a straight- or branched- chain alkyl or alkenyl group containing up to four carbon atoms optionally substituted by one or more chlorine, bromine or fluorine atoms;
a straight- or branched- chain alkynyl group containing three or four carbon atoms; or
a cyclopropyl group;
R¹² represents:-
the hydrogen atom;
a straight- or branched- chain alkyl or alkenyl group containing up to four carbon atoms optionally substituted by one or more chlorine, bromine or fluorine atoms;
a straight- or branched- chain alkynyl group containing three or four carbon atoms; or
a cyclopropyl group;
m represents two or three; and
n represents zero, one or two.

4. A compound according to claim 1 wherein:
R represents a methyl, ethyl, isopropyl, cyclopropyl or 1-methylcyclopropyl group;
R², R³ and R⁴ which may be the same or different, each represents:-
a hydrogen, bromine, chlorine or fluorine atom; or
a straight- or branched- chain alkyl or alkenyl group containing up to four carbon atoms optionally substituted by one or more chlorine, bromine or fluorine atoms;
a group selected from -COR⁷, -CO₂R⁶, -SR⁹-, -O(CH₂)ₘOR⁶, -OR⁶¹ and -N(R¹²)SO₂R⁸; or
a straight- or branched chain alkyl group containing up to four carbon atoms which is substituted by -OR⁶;
R⁵ represents the hydrogen atom;
R⁶, R⁷ and R⁹ which may be the same or different, each represents a straight- or branched- chain alkyl group containing up to three carbon atoms;
R⁶¹ represents:-
a straight- or branched- chain alkyl group containing up to four carbon atoms optionally substituted by one or more chlorine, bromine or fluorine atoms;
a straight- or branched- chain alkenyl or alkynyl group containing three or four carbon atoms; or
a cyclopropyl group;
R⁸ represents:-
a straight- or branched- chain alkyl group containing up to three carbon atoms optionally substituted by one or more chlorine, bromine, or fluorine atoms; or
an allyl group optionally substituted by one or more chlorine, fluorine or bromine atoms;
R¹² represents:-
the hydrogen atom;
a straight- or branched- chain alkyl group containing up to three carbon atoms optionally substituted by one or more chlorine, bromine, or fluorine atoms; or
an allyl group optionally substituted by one or more chlorine, fluorine or bromine atoms; and
m represents two or three.

5. A compound according to claim 1 wherein:
R is cyclopropyl, R¹ is hydrogen, R² is -N(CH₃)SO₂CH₃, R³ is hydrogen, R⁴ is nitro and R⁵ is hydrogen;
R is cyclopropyl, R¹ is hydrogen, R² is chloro, R³ is hydrogen, R⁴ is -N(CH₃)SO₂CH₃ and R⁵ is hydrogen;
R is cyclopropyl, R¹ is hydrogen, R² is nitro, R³ is hydrogen, R⁴ is -N(CH₃)SO₂CH₃ and R⁵ is hydrogen;
R is cyclopropyl, R¹ is hydrogen, R² is -N(CH₃)SO₂CH₃, R³ is hydrogen, R⁴ is chloro and R⁵ is hydrogen;
R is cyclopropyl, R¹ is hydrogen, R² is -N(CH₃)SO₂CH₃, R³ is hydrogen, R⁴ is methyl and R⁵ is hydrogen;
R is cyclopropyl, R¹ is hydrogen, R² is chloro, R³ is hydrogen, R⁴ is -NHSO₂CH₃ and R⁵ is hydrogen;
R is cyclopropyl, R¹ is hydrogen, R² is -N(CH₂CH₃)SO₂CH₃, R³ is hydrogen, R⁴ is chloro and R⁵ is hydrogen;
R is cyclopropyl, R¹ is hydrogen, R² is -N(CH₃)SO₂CH₃, R³ is hydrogen, R⁴ is -OCF₃ and R⁵ is hydrogen;
R is cyclopropyl, R¹ is hydrogen, R² is -N(CH₃)SO₂CH₃, R³ and R⁴ are hydrogen and R⁵ is chlorine;
R is cyclopropyl, R¹ is hydrogen, R² is -N(CH₃)SO₂CH(CH₃)CH₃, R³ is hydrogen, R⁴ is chlorine and R⁵ is hydrogen;
R is cyclopropyl, R¹ is hydrogen, R² is -N(CH₃)SO₂CH₃, R³ is hydrogen and R⁴ and R⁵ are fluorine;
or an agriculturally acceptable salt, metal complex or enolic tautomeric form thereof.

6. A herbicidal composition comprising a herbicidally effective amount of a 2-cyano-1,3-dione derivative of formula (I) according to any one of claims 1 to 5, or a agriculturally acceptable salt, metal complex or enolic tautomeric form thereof, in association with an agriculturally acceptable diluent or carrier and/or surface active agent.

7. A herbicidal composition according to claim 6 in the form of an aqueous suspension concentrate, a wettable powder, a water soluble or water dispersible powder, a liquid water soluble concentrate, a liquid emulsifiable suspension concentrate, a granule or an emulsifiable concentrate.

8. A method for controlling the growth of weeds at a locus which comprises applying to the locus a herbicidally effective amount of a 2-cyano-1,3-dione derivative of formula (I) as defined in any one of claims 1 to 5 or an agriculturally acceptable salt, metal complex or enolic tautomeric form thereof.

9. A method according to claim 8 in which the locus is an area used, or to be used, for growing of crops and the compound is applied at an application rate from 0.01 kg to 4.0 kg per hectare.

10. A process for the preparation of a 2-cyano-1,3-dione derivative of formula (I) as defined in claim 1 which comprises:
(a) reacting a compound of formula (II): 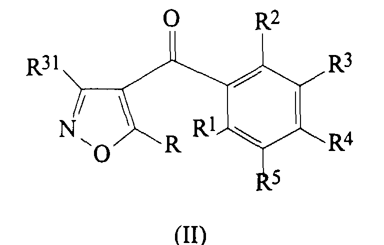 wherein R, R¹, R², R³, R⁴ and R⁵ are as defined in claim 1 and R³¹ represents the hydrogen atom or an acyl group, with a base;
(b) subjecting a compound of formula (II) above wherein R³¹ represents an ester, amide or nitrile, to acidic or basic hydrolysis;
(c) reacting a compound of formula (III): 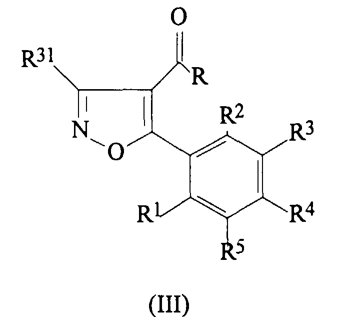 wherein R, R¹, R², R³, R⁴and R⁵ are as defined in claim 1 and R³¹ is hydrogen or an acyl group, with a base;
(d) subjecting a compound of formula (III) above wherein R³¹ represents an ester, amide or nitrile, to acidic or basic hydrolysis;
(e) reacting a benzoyl chloride of formula (IV): 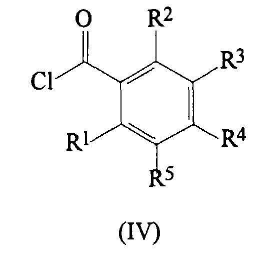 wherein R¹ R², R³, R⁴ and R⁵ are as defined in claim 1, with a beta-ketonitrile of formula(V): 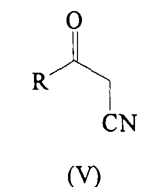 wherein R is as defined in claim 1;
(f) reacting an acid chloride of formula (VI): 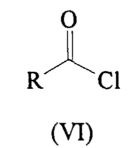 wherein R is as defined in claim 1, with a beta-ketonitrile of formula (VII): 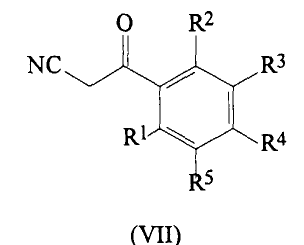 wherein R¹, R², R³, R⁴ and R⁵ are as defined in claim 1;
(g) reacting a benzoyl chloride of formula (IV) above with a beta-ketonitrile of formula (V) in the presence of a mild base to form an intermediate of formula (VIII): 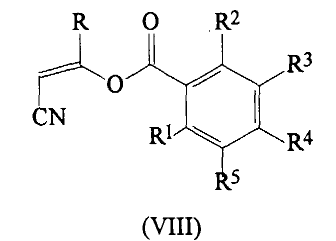 wherein R, R¹, R², R³, R⁴ and R⁵ are as defined in claim 1, followed by effecting rearrangement of the intermediate of formula (VIII) in the presence of a catalyst;
(h) reacting an acid chloride of formula (VI) as defined above with a beta-ketonitrile of formula (VII) as defined above in the presence of a mild base to form an intermediate of formula (IX): 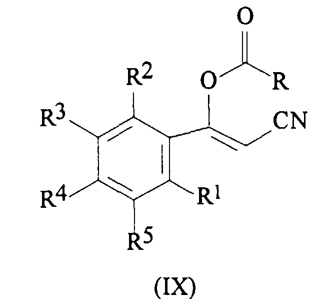 wherein R, R¹, R², R³, R⁴ and R⁵ are as defined in claim 1, followed by effecting rearrangement of the intermediate of formula (IX) in the presence of a catalyst;
optionally followed by the conversion of the compound thus obtained into an agriculturally acceptable salt or metal complex thereof.

## Patentansprüche

1. 2-Cyano-1,3-dion-Derivat der Formel 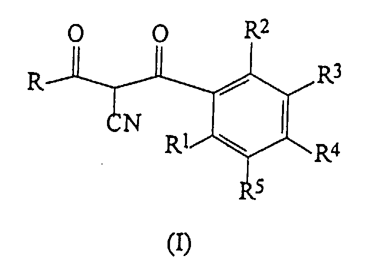 in der:
R:
für eine gerad- oder verzweigtkettige Alkylgruppe, die bis zu sechs Kohlenstoffatome enthält und gegebenenfalls durch ein oder mehrere Halogenatome substituiert ist, oder
für eine Cycloalkylgruppe, die drei bis sechs Kohlenstoffatome enthält und gegebenenfalls durch eine oder mehrere Gruppen R⁷ substituiert ist, steht;
R¹ für das Wasserstoffatom steht;
R², R³, R⁴ und R⁵, die gleich oder verschieden sein können, jeweils:
für das Wasserstoffatom,
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist,
für eine gerad- oder verzweigtkettige Alkylgruppe, die bis zu sechs Kohlenstoffatome enthält und durch eine Gruppe -OR⁶ substituiert ist,
für ein Halogenatom,
für Phenyl, das gegebenenfalls durch eine bis drei Gruppen R²¹, die gleich oder verschieden sein können, substituiert ist, für eine Gruppe, ausgewählt aus -COR⁷, Nitro, Cyano, -CO₂R⁶, -S(O)ₙR⁹, -O(CH₂)ₘOR⁶, -N(R¹²)SO₂R⁸, -CONR¹⁰R¹⁵ und -OR⁶¹, stehen, mit der Maßgabe, daß mindestens eine der Gruppen R² bis R⁵ für -N(R¹²)SO₂R⁸ steht;
R⁶:
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist, oder
für eine Cycloalkylgruppe, die drei bis sechs Kohlenstoffatome enthält, steht;
R⁶¹:
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist,
für eine Cycloalkylgruppe, die drei bis sechs Kohlenstoffatome enthält, oder
für Phenyl, das gegebenenfalls durch eine bis fünf Gruppen R²¹, die gleich oder verschieden sein können, substituiert ist, steht;
R⁷:
für eine gerad- oder verzweigtkettige Alkylgruppe, die bis zu sechs Kohlenstoffatome enthält und gegebenenfalls durch ein oder mehrere Halogenatome substituiert ist, oder
für eine Cycloalkylgruppe, die drei bis sechs Kohlenstoffatome enthält, steht;
R⁸:
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist,
für eine Cycloalkylgruppe, die drei bis sechs Kohlenstoffatome enthält, oder
für Phenyl, das gegebenenfalls durch eine bis fünf Gruppen R²¹, die gleich oder verschieden sein können, substituiert ist, oder für -NR¹⁰R¹¹ steht;
R⁹ :
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist,
für eine Cycloalkylgruppe, die drei bis sechs Kohlenstoffatome enthält, oder
für Phenyl, das gegebenenfalls durch eine bis fünf Gruppen R²¹, die gleich oder verschieden sein können, substituiert ist, steht;
R¹⁰ für Wasserstoff oder eine gerad- oder verzweigtkettige Alkylgruppe, die bis zu sechs Kohlenstoffatome enthält und gegebenenfalls durch ein oder mehrere Halogenatome substituiert ist, steht;
R¹¹:
für eine gerad- oder verzweigtkettige Alkylgruppe, die bis zu sechs Kohlenstoffatome enthält und gegebenenfalls durch ein oder mehrere Halogenatome substituiert ist, steht;
wenn R¹⁰ und R¹¹ Teil einer Gruppe -NR¹⁰R¹¹ sind, können sie zusammen mit dem Stickstoff, an den sie gebunden sind, einen fünf- oder sechsgliedrigen Ring bilden, der gegebenenfalls ein zusätzliches Heteroatom, das Sauerstoff oder Stickstoff ist, in dem Ring aufweist, wobei der Ring gegebenenfalls durch eine oder mehrere Alkylgruppen, die bis zu drei Kohlenstoffatome enthalten, substituiert ist;
R¹²:
für das Wasserstoffatom oder
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist,
für eine Cycloalkylgruppe, die drei bis sechs Kohlenstoffatome enthält,
für Phenyl, das gegebenenfalls durch eine bis fünf Gruppen R²¹, die gleich oder verschieden sein können, substituiert ist,
oder für eine Gruppe -OR¹⁷ steht;
R¹⁵ für eine Gruppe, ausgewählt aus R⁷ und -OR¹⁷, steht; wenn R¹⁰ und R¹⁵ Teil einer Gruppe -CONR¹⁰R¹⁵ sind, können sie zusammen mit dem Stickstoff, an den sie gebunden sind, einen fünf- oder sechsgliedrigen Ring bilden, der gegebenenfalls ein zusätzliches Heteroatom, das Sauerstoff oder Stickstoff ist, in dem Ring aufweist, wobei der Ring gegebenenfalls durch eine oder mehrere Alkylgruppen, die bis zu drei Kohlenstoffatome enthalten, substituiert ist;
R¹⁷ für eine gerad- oder verzweigtkettige Alkylgruppe, die bis zu sechs Kohlenstoffatome enthält, steht;
R²¹:
für ein Halogenatom,
für eine gerad- oder verzweigtkettige Alkylgruppe, die bis zu drei Kohlenstoffatome enthält und gegebenenfalls durch ein oder mehrere Halogenatome substituiert ist, oder
für eine Gruppe, ausgewählt aus Nitro, Cyano, -S(O)ₙR⁷ und -OR⁷, steht;
m für eins, zwei oder drei steht und
n für Null, eins oder zwei steht,
oder ein landwirtschaftlich annehmbares Salz, ein landwirtschaftlich annehmbarer Metallkomplex oder eine landwirtschaftlich annehmbare enolische tautomere Form davon.

2. Verbindung nach Anspruch 1, wobei:
R:
für eine gerad- oder verzweigtkettige Alkylgruppe, die bis zu sechs Kohlenstoffatome enthält und gegebenenfalls durch ein oder mehrere Halogenatome substituiert ist, oder
für eine Cycloalkylgruppe, die drei bis sechs Kohlenstoffatome enthält und gegebenenfalls durch eine oder mehrere Methylgruppen substituiert ist, steht,
R², R³, R⁴ und R⁵, die gleich oder verschieden sein können, jeweils:
für ein Wasserstoff- oder Halogenatom,
für eine gerad- oder verzweigtkettige Alkyl-, Alkenyl- oder Alkinylgruppe, die bis zu sechs Kohlenstoffatome enthält und gegebenenfalls durch ein oder mehrere Halogenatome substituiert ist,
für eine gerad- oder verzweigtkettige Alkylgruppe, die bis zu sechs Kohlenstoffatome enthält und durch eine Gruppe -OR⁶ substituiert ist,
für Phenyl, das gegebenenfalls durch eine bis drei Gruppen R²¹, die gleich oder verschieden sein können, substituiert ist, oder für eine Gruppe, ausgewählt aus -COR⁷, Cyano, Nitro, -CO₂R⁶, -S(O)ₙR⁹, -O(CH₂)ₘOR⁶, -N(R¹²)SO₂R⁸ und -OR⁶¹, stehen;
R⁶ und R⁷, die gleich oder verschieden sein können, jeweils:
für eine gerad- oder verzweigtkettige Alkylgruppe, die bis zu sechs Kohlenstoffatome enthält und gegebenenfalls durch ein oder mehrere Halogenatome substituiert ist, oder
für eine Cycloalkylgruppe, die drei bis sechs Kohlenstoffatome enthält, stehen;
R⁶¹ und R⁹, die gleich oder verschieden sein können, jeweils:
für eine gerad- oder verzweigtkettige Alkyl- oder Alkenylgruppe, die bis zu sechs Kohlenstoffatome enthält und gegebenenfalls durch ein oder mehrere Halogenatome substituiert ist,
für eine gerad- oder verzweigtkettige Alkinylgruppe, die drei bis sechs Kohlenstoffatome enthält,
für eine Cycloalkylgruppe, die drei bis sechs Kohlenstoffatome enthält, stehen;
R⁸:
für eine gerad- oder verzweigtkettige Alkyl- oder Alkenylgruppe, die bis zu sechs Kohlenstoffatome enthält und gegebenenfalls durch ein oder mehrere Halogenatome substituiert ist,
für eine gerad- oder verzweigtkettige Alkinylgruppe, die drei bis sechs Kohlenstoffatome enthält,
für eine Cycloalkylgruppe, die drei bis sechs Kohlenstoffatome enthält, oder
für Phenyl, das gegebenenfalls durch eine bis drei Gruppen R²¹, die gleich oder verschieden sein können, substituiert ist, steht;
R¹²:
für das Wasserstoffatom oder
für eine gerad- oder verzweigtkettige Alkyl- oder Alkenylgruppe, die bis zu sechs Kohlenstoffatome enthält und gegebenenfalls durch ein oder mehrere Halogenatome substituiert ist,
für eine gerad- oder verzweigtkettige Alkinylgruppe, die drei bis sechs Kohlenstoffatome enthält, oder
für eine Cycloalkylgruppe, die drei bis sechs Kohlenstoffatome enthält, steht;
R²¹:
für ein Halogenatom,
für eine gerad- oder verzweigtkettige Alkylgruppe, die bis zu drei Kohlenstoffatome enthält und gegebenenfalls durch ein oder mehrere Halogenatome substituiert ist, oder
für eine Gruppe, ausgewählt aus Nitro, Cyano, -S(O)ₙR⁷ oder -OR⁷, steht;
m für zwei oder drei steht und
n für Null, eins oder zwei steht.

3. Verbindung nach Anspruch 1, wobei:
R:
für eine gerad- oder verzweigtkettige Alkylgruppe, die bis zu drei Kohlenstoffatome enthält, oder
für eine Cycloalkylgruppe, die drei oder vier Kohlenstoffatome enthält und gegebenenfalls durch eine Methylgruppe substituiert ist, steht;
R², R³ und R⁴, die gleich oder verschieden sein können, jeweils:
für ein Wasserstoff-, Chlor-, Brom- oder Fluoratom oder
für eine gerad- oder verzweigtkettige Alkyl-, Alkenyl- oder Alkinylgruppe, die bis zu vier Kohlenstoffatome enthält und gegebenenfalls durch ein oder mehrere Halogenatome substituiert ist,
für eine gerad- oder verzweigtkettige Alkylgruppe, die bis zu vier Kohlenstoffatome enthält und durch eine Gruppe -OR⁶ substituiert ist, oder
für eine Gruppe, ausgewählt aus -COR⁷, -CO₂R⁶, -S(O)ₙR⁹, -O(CH₂)ₘOR⁶, -N(R¹²)SO₂R⁸ und -OR⁶¹, stehen;
R⁵ für das Wasserstoffatom steht;
R⁶, R⁷ und R⁹, die gleich oder verschieden sein können, jeweils:
für eine gerad- oder verzweigtkettige Alkylgruppe, die bis zu vier Kohlenstoffatome enthält und gegebenenfalls durch ein oder mehrere Chlor-, Brom- oder Fluoratome substituiert ist, oder für eine Cyclopropylgruppe stehen;
R⁶¹ und R⁸, die gleich oder verschieden sein können, jeweils:
für eine gerad- oder verzweigtkettige Alkyl- oder Alkenylgruppe, die bis zu vier Kohlenstoffatome enthält und gegebenenfalls durch ein oder mehrere Chlor-, Brom- oder Fluoratome substituiert ist,
für eine gerad- oder verzweigtkettige Alkinylgruppe, die drei oder vier Kohlenstoffatome enthält, oder für eine Cyclopropylgruppe stehen;
R¹²:
für das Wasserstoffatom oder
für eine gerad- oder verzweigtkettige Alkyl- oder Alkenylgruppe, die bis zu vier Kohlenstoffatome enthält und gegebenenfalls durch ein oder mehrere Chlor-, Brom- oder Fluoratome substituiert ist,
für eine gerad- oder verzweigtkettige Alkinylgruppe, die drei oder vier Kohlenstoffatome enthält, oder
für eine Cyclopropylgruppe steht;
m für zwei oder drei steht und
n für Null, eins oder zwei steht.

4. Verbindung nach Anspruch 1, wobei
R für eine Methyl-, Ethyl-, Isopropyl-, Cyclopropyl- oder 1-Methylcyclopropylgruppe steht;
R², R³ und R⁴, die gleich oder verschieden sein können, jeweils:
für ein Wasserstoff-, Brom-, Chlor- oder Fluoratom oder für eine gerad- oder verzweigtkettige Alkyl- oder Alkenylgruppe, die bis zu vier Kohlenstoffatome enthält und gegebenenfalls durch ein oder mehrere Chlor-, Brom- oder Fluoratome substituiert ist,
für eine Gruppe, ausgewählt aus -COR⁷, -CO₂R⁶, -SR⁹-, -O(CH₂)ₘOR⁶, -OR⁶¹ und -N(R¹²)SO₂R⁸, oder
für eine gerad- oder verzweigtkettige Alkylgruppe, die bis zu vier Kohlenstoffatome enthält und durch -OR⁶ substituiert ist, stehen;
R⁵ für das Wasserstoffatom steht;
R⁶, R⁷ und R⁹, die gleich oder verschieden sein können, jeweils für eine gerad- oder verzweigtkettige Alkylgruppe, die bis zu drei Kohlenstoffatome enthält, stehen;
R⁶¹:
für eine gerad- oder verzweigtkettige Alkylgruppe, die bis zu vier Kohlenstoffatome enthält und gegebenenfalls durch ein oder mehrere Chlor-, Brom- oder Fluoratome substituiert ist,
für eine gerad- oder verzweigtkettige Alkenyl- oder Alkinylgruppe, die drei oder vier Kohlenstoffatome enthält, oder für eine Cyclopropylgruppe steht;
R⁸:
für eine gerad- oder verzweigtkettige Alkylgruppe, die bis zu drei Kohlenstoffatome enthält und gegebenenfalls durch ein oder mehrere Chlor-, Brom- oder Fluoratome substituiert ist, oder für eine Allylgruppe, die gegebenenfalls durch ein oder mehrere Chlor-, Fluor- oder Bromatome substituiert ist, steht; R¹²:
für das Wasserstoffatom,
für eine gerad- oder verzweigtkettige Alkylgruppe, die bis zu drei Kohlenstoffatome enthält und gegebenenfalls durch ein oder mehrere Chlor-, Brom- oder Fluoratome substituiert ist, oder für eine Allylgruppe, die gegebenenfalls durch ein oder mehrere Chlor-, Fluor- oder Bromatome substituiert ist, steht und m für zwei oder drei steht.

5. Verbindung nach Anspruch 1, wobei
R Cyclopropyl ist, R¹ Wasserstoff ist, R² -N(CH₃)SO₂CH₃ ist, R³ Wasserstoff ist, R⁴ Nitro ist und R⁵ Wasserstoff ist;
R Cyclopropyl ist, R¹ Wasserstoff ist, R² Chlor ist, R³ Wasserstoff ist, R⁴ -N(CH₃)SO₂CH₃ ist und R⁵ Wasserstoff ist;
R Cyclopropyl ist, R¹ Wasserstoff ist, R² Nitro ist, R³ Wasserstoff ist, R⁴ -N(CH₃)SO₂CH₃ ist und R⁵ Wasserstoff ist;
R Cyclopropyl ist, R¹ Wasserstoff ist, R² -N(CH₃)SO₂CH₃ ist, R³ Wasserstoff ist, R⁴ Chlor ist und R⁵ Wasserstoff ist;
R Cyclopropyl ist, R¹ Wasserstoff ist, R² -N(CH₃)SO₂CH₃ ist, R³ Wasserstoff ist, R⁴ Methyl ist und R⁵ Wasserstoff ist;
R Cyclopropyl ist, R¹ Wasserstoff ist, R² Chlor ist, R³ Wasserstoff ist, R⁴ -NHSO₂CH₃ ist und R⁵ Wasserstoff ist;
R Cyclopropyl ist, R¹ Wasserstoff ist, R² -N(CH₂CH₃)SO₂CH₃ ist, R³ Wasserstoff ist, R⁴ Chlor ist und R⁵ Wasserstoff ist;
R Cyclopropyl ist, R¹ Wasserstoff ist, R² -N(CH₃)SO₂CH₃ ist, R³ Wasserstoff ist, R⁴ -OCF3 ist und R⁵ Wasserstoff ist;
R Cyclopropyl ist, R¹ Wasserstoff ist, R² -N(CH₃)SO₂CH₃ ist, R³ und R⁴ Wasserstoff sind und R⁵ Chlor ist;
R Cyclopropyl ist, R¹ Wasserstoff ist, R² -N(CH₃)SO₂CH(CH₃)CH₃, R³ Wasserstoff ist, R⁴ Chlor ist und R⁵ Wasserstoff ist;
R Cyclopropyl ist, R¹ Wasserstoff ist, R² -N(CH₃)SO₂CH₃ ist, R³ Wasserstoff ist, R⁴ und R⁵ Fluor sind;
oder ein landwirtschaftlich annehmbares Salz, ein landwirtschaftlich annehmbarer Metallkomplex oder eine landwirtschaftlich annehmbare enolische tautomere Form davon.

6. Herbizide Zusammensetzung, umfassend eine herbizid wirksame Menge eines 2-Cyano-1,3-dion-Derivats der Formel (I) nach einem der Ansprüche 1 bis 5 oder eines landwirtschaftlich annehmbaren Salzes, eines landwirtschaftlich annehmbaren Metallkomplexes oder einer landwirtschaftlich annehmbaren enolischen tautomeren Form davon in Verbindung mit einem landwirtschaftlich annehmbaren Verdünnungsmittel oder Träger und/oder grenzflächenaktiven Mittel.

7. Herbizide Zusammensetzung nach Anspruch 6 in Form eines wäßrigen Suspensionskonzentrats, eines benetzbaren Pulvers, eines wasserlöslichen oder in Wasser dispergierbaren Pulvers, eines flüssigen, wasserlöslichen Konzentrats, eines flüssigen, emulgierbaren Suspensionskonzentrats, eines Granulats oder eines emulgierbaren Konzentrats.

8. Verfahren zum Kontrollieren des Wachstums von Unkräutern an einem Ort, welches umfaßt, auf den Ort eine herbizid wirksame Menge eines 2-Cyano-1,3-dion-Derivats der Formel (I), wie in einem der Ansprüche 1 bis 5 definiert, oder eines landwirtschaftlich annehmbaren Salzes, eines landwirtschaftlich annehmbaren Metallkomplexes oder einer landwirtschaftlich annehmbaren enolischen tautomeren Form davon auszubringen.

9. Verfahren nach Anspruch 8, wobei der Ort eine Fläche ist, die zum Anbauen von Feldfrüchten verwendet wird oder verwendet werden soll, und die Verbindung in einer Einsatzmenge von 0,01 kg bis 4,0 kg pro Hektar ausgebracht wird.

10. Verfahren zur Herstellung eines 2-Cyano-1,3-dion-Derivats der Formel (I), wie in Anspruch 1 definiert, welches umfaßt:
(a) eine Verbindung der Formel (II) : 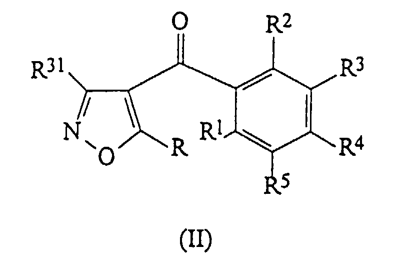 in der R, R¹, R², R³, R⁴ und R⁵ wie in Anspruch 1 definiert sind und R³¹ für das Wasserstoffatom oder eine Acylgruppe steht, mit einer Base umzusetzen;
(b) eine Verbindung der vorstehend angegebenen Formel (II), in der R³¹ für einen Ester, ein Amid oder Nitril steht, einer sauren oder basischen Hydrolyse zu unterwerfen;
(c) eine Verbindung der Formel (III): 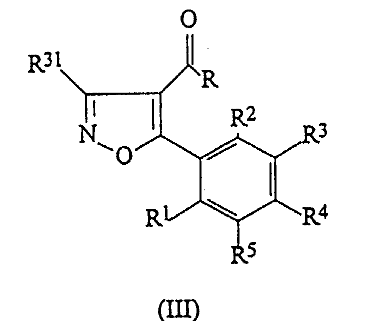 in der R, R¹, R², R³, R⁴ und R⁵ wie in Anspruch 1 definiert sind und R³¹ Wasserstoff oder eine Acylgruppe ist, mit einer Base umzusetzen;
(d) eine Verbindung der vorstehend angegebenen Formel (III), in der R³¹ für einen Ester, ein Amid oder Nitril steht, einer sauren oder basischen Hydrolyse zu unterwerfen;
(e) ein Benzoylchlorid der Formel (IV) : 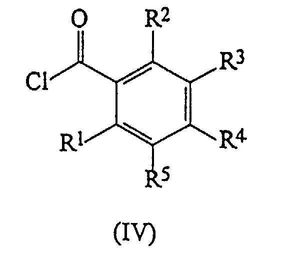 in der R¹, R², R³, R⁴ und R⁵ wie in Anspruch 1 definiert sind, mit einem β-Ketonitril der Formel (V); 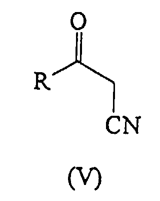 wobei R wie in Anspruch 1 definiert ist, umzusetzen;
(f) ein Säurechlorid der Formel (VI): 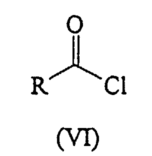 in der R wie in Anspruch 1 definiert ist, mit einem β-Ketonitril der Formel (VII): 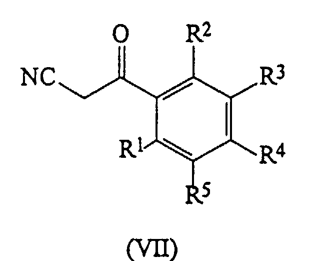 in der R¹, R², R³, R⁴ und R⁵ wie in Anspruch 1 definiert sind, umzusetzen;
(g) ein Benzoylchlorid der vorstehend angegebenen Formel (IV) mit einem β-Ketonitril der Formel (V) in Anwesenheit einer milden Base umzusetzen zur Bildung eines Zwischenprodukts der Formel (VIII): 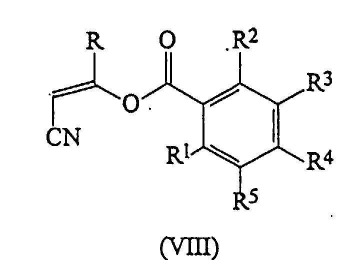 in der R, R¹, R², R³, R⁴ und R⁵ wie in Anspruch 1 definiert sind, gefolgt von einem Bewirken einer Umlagerung des Zwischenprodukts der Formel (VIII) in Anwesenheit eines Katalysators;
(h) Umsetzen eines Säurechlorids der Formel (VI), wie vorstehend definiert, mit einem β-Ketonitril der Formel (VII), wie vorstehend definiert, in Anwesenheit einer milden Base zur Bildung eines Zwischenprodukts der Formel (IX): 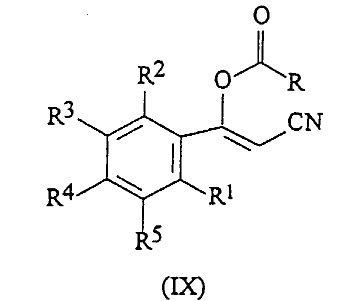 in der R, R¹, R², R³, R⁴ und R⁵ wie in Anspruch 1 definiert sind, gefolgt von einem Bewirken einer Umlagerung des Zwischenprodukts der Formel (IX) in Anwesenheit eines Katalysators;
gegebenenfalls gefolgt von der Umwandlung der so erhaltenen Verbindung in ein landwirtschaftlich annehmbares Salz oder einen landwirtschaftlich annehmbaren Metallkomplex davon.

## Revendications

1. Dérivé 2-cyano-1,3-dione de formule : 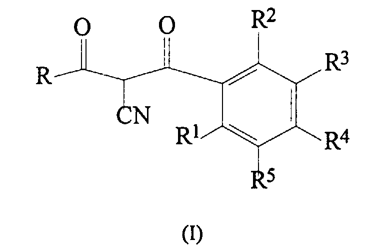 dans laquelle :
R représente : -
un radical alkyle linéaire ou ramifié contenant jusqu'à six atomes de carbone éventuellement substitué par un ou plusieurs atomes d'halogènes ; ou
un radical cycloalkyle contenant de trois à six atomes de carbone éventuellement substitué par un ou plusieurs radicaux R⁷ ;
R¹ représente l'atome d'hydrogène ;
R², R³, R⁴ et R⁵, qui peuvent être identiques ou différents, représentent chacun :-
l'atome d'hydrogène ;
un radical alkyle, alkényle ou alkynyle linéaire ou ramifié contenant jusqu'à six atomes de carbone éventuellement substitué par un ou plusieurs atomes d'halogènes ;
un radical alkyle linéaire ou ramifié contenant jusqu'à six atomes de carbone substitué par un radical -OR⁶ ;
un atome d'halogènes ;
un radical phényle substitué par un à trois radicaux R²¹ qui peuvent être identiques ou différents ;
un radical choisi parmi -COR⁷, nitro, cyano, -CO₂R⁶, -S(O)ₙR⁹, -O(CH₂)ₘOR⁶, -N(R¹²)SO₂R⁸, -CONR¹⁰R¹⁵ et -OR⁶¹ ;
avec la restriction que au moins un des radicaux R² à R⁵ représente -N(R¹²)SO₂R⁸;
R⁶ représente : -
un radical alkyle, alkényle ou alkynyle linéaire ou ramifié contenant jusqu'à six atomes de carbone éventuellement substitué par un ou plusieurs atomes d'halogènes ; ou
un radical cycloalkyle contenant de trois à six atomes de carbone ;
R⁶¹ représente : -
un radical alkyle, alkényle ou alkynyle linéaire ou ramifié contenant jusqu'à six atomes de carbone éventuellement substitué par un ou plusieurs atomes d'halogènes ;
un radical cycloalkyle contenant de trois à six atomes de carbone ;
ou un radical phényle éventuellement substitué par un à cinq radicaux R²¹ qui peuvent être identiques ou différents ;
R⁷ représente : -
un radical alkyle linéaire ou ramifié contenant jusqu'à six atomes de carbone éventuellement substitué par un ou plusieurs atomes d'halogènes ;
ou un radical cycloalkyle contenant de trois à six atomes de carbone ;
R⁸ représente : -
un radical alkyle, alkényle ou alkynyle linéaire ou ramifié contenant jusqu'à six atomes de carbone éventuellement substitué par un ou plusieurs atomes d'halogènes ;
un radical cycloalkyle contenant de trois à six atomes de carbone ;
un radical phényle éventuellement substitué par un à cinq radicaux R²¹ qui peuvent être identiques ou différents ; ou -NR¹⁰R¹¹ ;
R⁹ représente : -
un radical alkyle, alkényle ou alkynyle linéaire ou ramifié contenant jusqu'à six atomes de carbone éventuellement substitué par un ou plusieurs atomes d'halogènes ;
un radical cycloalkyle contenant de trois à six atomes de carbone ;
ou un radical phényle éventuellement substitué par un à cinq radicaux R²¹ qui peuvent être identiques ou différents ;
R¹⁰ représente l'atome d'hydrogène ou un radical alkyle linéaire ou ramifié contenant jusqu'à six atomes de carbone éventuellement substitué par un ou plusieurs atomes d'halogènes ;
R¹¹ représente :-
un radical alkyle linéaire ou ramifié contenant jusqu'à six atomes de carbone éventuellement substitué par un ou plusieurs atomes d'halogènes ;
lorsque R¹⁰ et R¹¹ font partie d'un radical -NR¹⁰R¹¹ ils peuvent former ensemble, avec l'atome d'azote auquel ils sont reliés, un noyau comportant cinq ou six chaînons possédant éventuellement un autre hétéréoatome dans la chaîne, l'oxygène ou l'azote, ce noyau étant éventuellement substitué par un ou plusieurs radicaux alkyle contenant jusqu'à trois atomes de carbone ;
R¹² représente :-
l'atome d'hydrogène ; ou
un radical alkyle, alkényle ou alkynyle linéaire ou ramifié contenant jusqu'à six atomes de carbone éventuellement substitué par un ou plusieurs atomes d'halogènes ;
un radical cycloalkyle contenant de trois à six atomes de carbone ;
un radical phényle éventuellement substitué par un à cinq radicaux R²¹ qui peuvent être identiques ou différents ; ou
un radical R¹⁷ ;
R¹⁵ représente un radical choisi parmi R⁷ et -OR¹⁷ ;
lorsque R¹⁰ et R¹⁵ font partie d'un radical -CONR¹⁰R¹⁵ ils peuvent former ensemble, avec l'atome d'azote auquel ils sont reliés, un noyau comportant cinq ou six chaînons possédant éventuellement un autre hétéréoatome dans la chaîne, l'oxygène ou l'azote, ce noyau étant éventuellement substitué par un ou plusieurs radicaux alkyle contenant jusqu'à trois atomes de carbone ;
R¹⁷ représente un radical alkyle linéaire ou ramifié contenant jusqu'à six atomes de carbone ;
R²¹ représente :-
un atome d'halogènes ;
un radical alkyle linéaire ou ramifié contenant jusqu'à trois atomes de carbone éventuellement substitué par un ou plusieurs atomes d'halogènes ;
ou un radical choisi parmi nitro, cyano, -S(O)ₙR⁷ et -OR⁷ ;
m représente un, deux ou trois ; et
n représente zéro, un ou deux
ou un sel, un complexe métallique ou une forme énolique tautomère de celui-ci, acceptables en agriculture.

2. Composé selon la revendication 1 dans laquelle :
R représente : -
un radical alkyle linéaire ou ramifié contenant jusqu'à six atomes de carbone éventuellement substitué par un ou plusieurs atomes d'halogènes ; ou
un radical cycloalkyle contenant de trois à six atomes de carbone éventuellement substitué par un ou plusieurs radicaux méthyle ;
R², R³, R⁴ et R⁵, qui peuvent être identiques ou différents, représentent chacun :-
l'atome d'hydrogène ou un atome d'halogènes ;
un radical alkyle, alkényle ou alkynyle linéaire ou ramifié contenant jusqu'à six atomes de carbone éventuellement substitué par un ou plusieurs atomes d'halogènes ;
un radical alkyle linéaire ou ramifié contenant jusqu'à six atomes de carbone substitué par un radical -OR⁶ ;
un radical phényle substitué par un à trois radicaux R²¹ qui peuvent être identiques ou différents ;
ou un radical choisi parmi -COR⁷, nitro, cyano, -CO₂R⁶, -S(O)ₙR⁹, -O(CH₂)ₘOR⁶, -N(R¹²)SO₂R⁸ et -OR⁶¹ ;
R⁶ et R⁷ qui peuvent être identiques ou différents, représentent chacun : -
un radical alkyle linéaire ou ramifié contenant jusqu'à six atomes de carbone éventuellement substitué par un ou plusieurs atomes d'halogènes ;
ou un radical cycloalkyle contenant de trois à six atomes de carbone ;
R⁶¹ et R⁹ qui peuvent être identiques ou différents, représentent chacun :-
un radical alkyle ou alkényle linéaire ou ramifié contenant jusqu'à six atomes de carbone éventuellement substitué par un ou plusieurs atomes d'halogènes ;
un radical alkynyle linéaire ou ramifié contenant de trois à six atomes de carbone ;
un radical cycloalkyle contenant de trois à six atomes de carbone ;
R⁸ représente : -
un radical alkyle ou alkényle linéaire ou ramifié contenant jusqu'à six atomes de carbone éventuellement substitué par un ou plusieurs atomes d'halogènes ;
un radical alkynyle linéaire ou ramifié contenant de trois à six atomes de carbone ;
un radical cycloalkyle contenant de trois à six atomes de carbone ; ou
un radical phényle éventuellement substitué par un à trois radicaux R²¹ qui peuvent être identiques ou différents ;
R¹² représente :-
l'atome d'hydrogène ; ou
un radical alkyle ou alkényle linéaire ou ramifié contenant jusqu'à six atomes de carbone éventuellement substitué par un ou plusieurs atomes d'halogènes ;
un radical alkynyle linéaire ou ramifié contenant de trois à six atomes de carbone ;
ou un radical cycloalkyle contenant de trois à six atomes de carbone ;
R²¹ représente :-
un atome d'halogènes ;
un radical alkyle linéaire ou ramifié contenant jusqu'à trois atomes de carbone éventuellement substitué par un ou plusieurs atomes d'halogènes ; ou
un radical choisi parmi nitro, cyano, -S(O)ₙR⁷ et -OR⁷ ;
m représente deux ou trois ; et
n représente zéro, un ou deux.

3. Composé selon la revendication 1 dans laquelle :
R représente : -
un radical alkyle linéaire ou ramifié contenant jusqu'à trois atomes de carbone ; ou
un radical cycloalkyle contenant trois ou quatre atomes de carbone éventuellement substitué par un radical méthyle ;
R², R³ et R⁴, qui peuvent être identiques ou différents, représentent chacun : -
un atome d'hydrogène, de chlore, de brome ou de fluor ; ou
un radical alkyle, alkényle ou alkynyle linéaire ou ramifié contenant jusqu'à quatre atomes de carbone éventuellement substitué par un ou plusieurs atomes d'halogènes ;
un radical alkyle linéaire ou ramifié contenant jusqu'à quatre atomes de carbone substitué par un radical -OR⁶ ; ou
un radical choisi parmi -COR⁷, -CO₂R⁶, -S(O)ₙR⁹, -O(CH₂)ₘOR⁶, -N(R¹²)SO₂R⁸ et -OR⁶¹ ;
R⁵ représente l'atome d'hydrogène ;
R⁶, R⁷ et R⁹ qui peuvent être identiques ou différents, représentent chacun : -
un radical alkyle linéaire ou ramifié contenant jusqu'à quatre atomes de carbone éventuellement substitué par un ou plusieurs atomes de chlore, de brome ou de fluor ; ou
un radical cyclopropyle ;
R⁶¹ et R⁸ qui peuvent être identiques ou différents, représentent chacun :-
un radical alkyle ou alkényle linéaire ou ramifié contenant jusqu'à quatre atomes de carbone éventuellement substitué par un ou plusieurs atomes de chlore, de brome ou de fluor ;
un radical alkynyle linéaire ou ramifié contenant trois ou quatre atomes de carbone ; ou
un radical cyclopropyle ;
R¹² représente :-
l'atome d'hydrogène ;
un radical alkyle ou alkényle linéaire ou ramifié contenant jusqu'à quatre atomes de carbone éventuellement substitué par un ou plusieurs atomes de chlore, de brome ou de fluor ;
un radical alkynyle linéaire ou ramifié contenant trois ou quatre atomes de carbone ; ou
un radical cyclopropyle ;
m représente deux ou trois ; et
n représente zéro, un ou deux.

4. Composé selon la revendication 1 dans laquelle :
R représente un radical méthyle, éthyle, isopropyle, cyclopropyle ou 1-méthylcyclopropyle ;
R², R³ et R⁴, qui peuvent être identiques ou différents, représentent chacun : -
un atome d'hydrogène, de brome, de chlore ou de fluor ; ou
un radical alkyle ou alkényle linéaire ou ramifié contenant jusqu'à quatre atomes de carbone éventuellement substitué par un ou plusieurs atomes de chlore, de brome ou de fluor ;
un radical choisi parmi -COR⁷, -CO₂R⁶, -SR⁹, -O(CH₂)ₘOR⁶, -OR⁶¹ et -N(R¹²)SO₂R⁸ ; ou
un radical alkyle linéaire ou ramifié contenant jusqu'à quatre atomes de carbone substitué par un radical -OR⁶ ;
R⁵ représente l'atome d'hydrogène ;
R⁶, R⁷ et R⁹ qui peuvent être identiques ou différents, représentent chacun un radical alkyle linéaire ou ramifié contenant jusqu'à trois atomes de carbone ;
R⁶¹ représente :-
un radical alkyle linéaire ou ramifié contenant jusqu'à quatre atomes de carbone éventuellement substitué par un ou plusieurs atomes de chlore, de brome ou de fluor ;
un radical alkényle ou alkynyle linéaire ou ramifié contenant trois ou quatre atomes de carbone ; ou
un radical cyclopropyle ;
R⁸ représente : -
un radical alkyle linéaire ou ramifié contenant jusqu'à trois atomes de carbone éventuellement substitué par un ou plusieurs atomes de chlore, de brome ou de fluor ;
un radical allyle éventuellement substitué par un ou plusieurs atomes de chlore, de brome ou de fluor ;
R¹² représente :-
l'atome d'hydrogène ;
un radical alkyle ou alkényle linéaire ou ramifié contenant jusqu'à trois atomes de carbone éventuellement substitué par un ou plusieurs atomes de chlore, de brome ou de fluor ; ou
un radical allyle éventuellement substitué par un ou plusieurs atomes de chlore, de brome ou de fluor ; et
m représente deux ou trois.

5. Composé selon la revendication 1 dans laquelle :
R représente cyclopropyle, R¹ représente l'atome d'hydrogène, R² représente -N(CH₃)SO₂CH₃, R³ représente l'atome d'hydrogène, R⁴ représente nitro et R⁵ représente l'atome d'hydrogène ;
R représente cyclopropyle, R¹ représente l'atome d'hydrogène, R² représente l'atome de chlore, R³ représente l'atome d'hydrogène, R⁴ représente -N(CH₃)SO₂CH₃ et R⁵ représente l'atome d'hydrogène ;
R représente cyclopropyle, R¹ représente l'atome d'hydrogène, R² représente nitro, R³ représente l'atome d'hydrogène, R⁴ représente -N(CH₃)SO₂CH₃ et R⁵ représente l'atome d'hydrogène ;
R représente cyclopropyle, R¹ représente l'atome d'hydrogène, R² représente -N(CH₃)SO₂CH₃, R³ représente l'atome d'hydrogène, R⁴ représente l'atome de chlore et R⁵ représente l'atome d'hydrogène ;
R représente cyclopropyle, R¹ représente l'atome d'hydrogène, R² représente -N(CH₃)SO₂CH₃, R³ représente l'atome d'hydrogène, R⁴ représente méthyle et R⁵ représente l'atome d'hydrogène ;
R représente cyclopropyle, R¹ représente l'atome d'hydrogène, R² représente l'atome de chlore, R³ représente l'atome d'hydrogène, R⁴ représente -NHSO₂CH₃ et R⁵ représente l'atome d'hydrogène ;
R représente cyclopropyle, R¹ représente l'atome d'hydrogène, R² représente -N(CH₂CH₃)SO₂CH₃, R³ représente l'atome d'hydrogène, R⁴ représente l'atome de chlore et R⁵ représente l'atome d'hydrogène ;
R représente cyclopropyle, R¹ représente l'atome d'hydrogène, R² représente -N(CH₃)SO₂CH₃, R³ représente l'atome d'hydrogène, R⁴ représente -OCF₃ et R⁵ représente l'atome d'hydrogène ;
R représente cyclopropyle, R¹ représente l'atome d'hydrogène, R² représente -N(CH₃)SO₂CH₃, R³ et R⁴ représentent l'atome d'hydrogène et R⁵ représente l'atome de chlore ;
R représente cyclopropyle, R¹ représente l'atome d'hydrogène, R² représente -N(CH₃)SO₂CH(CH₃)CH₃, R³ représente l'atome d'hydrogène, R⁴ représente l'atome de chlore et R⁵ représente l'atome d'hydrogène;
R représente cyclopropyle, R¹ représente l'atome d'hydrogène, R² représente -N(CH₃)SO₂CH₃, R³ représente l'atome d'hydrogène et R⁴ et R⁵ représentent l'atome de fluor ;
ou un sel, un complexe métallique ou une forme énolique tautomère de celui-ci, acceptables en agriculture.

6. Composition herbicide comprenant une quantité efficace du point de vue herbicide d'un dérivé 2-cyano-1,3-dione de formule (I) selon l'une quelconque des revendications 1 à 5, ou un sel, un complexe métallique ou une forme énolique tautomère de celui-ci acceptables en agriculture, en association avec un diluant ou un support et/ou un agent tensio-actif acceptables en agriculture.

7. Composition herbicide selon la revendication 6 sous forme de suspension concentrée aqueuse, poudre mouillable, poudre soluble dans l'eau ou dispersable dans l'eau, concentré liquide soluble dans l'eau, suspension concentrée émulsifiable liquide, granulés ou concentré émulsifiable.

8. Méthode de contrôle de la croissance des mauvaises herbes en un lieu qui comprend l'application en ce lieu d'une quantité efficace du point de vue herbicide d'un dérivé 2-cyano-1,3-dione de formule (I) telle que définie dans l'une quelconque des revendications 1 à 5, ou un sel, un complexe métallique ou une forme énolique tautomère de celui-ci acceptables en agriculture.

9. Méthode selon la revendication 8 dans laquelle le lieu est une zone utilisée, ou devant être utilisée, pour la croissance de cultures et dans laquelle le composé est appliqué à une dose comprise entre 0,01 kg et 4,0 kg par hectare.

10. Procédé de préparation d'un dérivé 2-cyano-1,3-dione de formule (I) telle que définie dans la revendication 1 qui comprend:
(a) réaction d'un composé de formule (II): 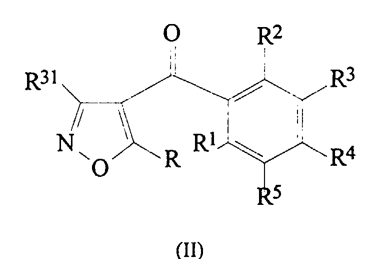 dans laquelle R, R¹, R², R³, R⁴ et R⁵ sont tels que définis dans la revendication 1 et R³¹ représente l'atome d'hydrogène ou un radical acyle, avec une base ;
(b) soumission d'un composé de formule (II) ci-dessus dans laquelle R³¹ représente un ester, une amide ou un nitrile, à une hydrolyse acide ou basique ;
(c) réaction d'un composé de formule (III): 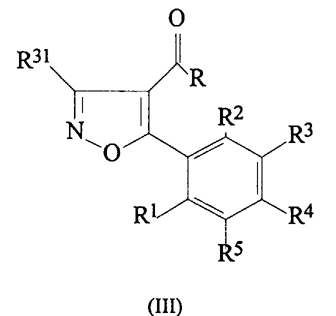 dans laquelle R, R¹, R², R³, R⁴ et R⁵ sont tels que définis dans la revendication 1 et R³¹ représente l'atome d'hydrogène ou un radical acyle, avec une base ;
(d) soumission d'un composé de formule (III) ci-dessus dans laquelle R³¹ représente un ester, une amide ou un nitrile, à une hydrolyse acide ou basique ;
(e) réaction d'un chlorure de benzoyle de formule (IV): 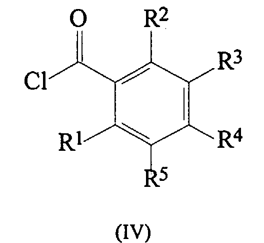 dans laquelle R¹, R², R³, R⁴ et R⁵ sont tels que définis dans la revendication 1, avec un béta-cétonitrile de formule (V): 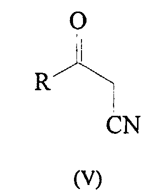 dans laquelle R est tel que défini dans la revendication 1 ;
(f) réaction d'un chlorure d'acide de formule (VI): 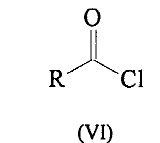 dans laquelle R est tel que défini dans la revendication 1, avec un béta-cétonitrile de formule (VII): 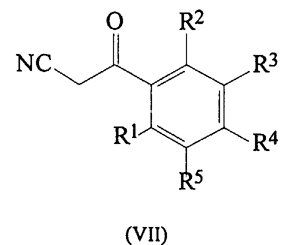 dans laquelle R¹, R², R³, R⁴ et R⁵ sont tels que définis dans la revendication 1;
(g) réaction d'un chlorure de benzoyle de formule (IV) ci-dessus avec un béta-cétonitrile de formule (V) en présence d'une base faible pour former un intermédiaire de formule (VIII): 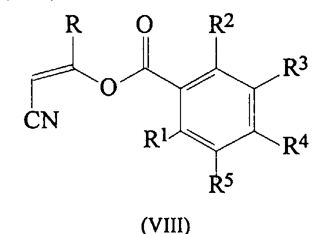 dans laquelle R, R¹, R², R³, R⁴ et R⁵ sont tels que définis dans la revendication 1, suivie de la réalisation du réarrangement de l'intermédiaire de formule (VIII) en présence d'un catalyseur ;
(h) réaction d'un chlorure d'acide de formule (VI) telle que définie précédemment avec un béta-cétonitrile de formule (VII) telle que définie précédemment en présence d'une base faible pour former un intermédiaire de formule (IX): 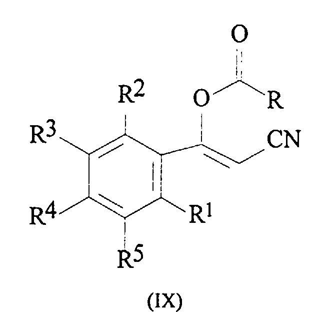 dans laquelle R, R¹, R², R³, R⁴ et R⁵ sont tels que définis dans la revendication 1, suivie de la réalisation du réarrangement de l'intermédiaire de formule (IX) en présence d'un catalyseur ;
éventuellement suivies de la conversion du composé ainsi obtenu en un sel ou un complexe métallique de celui-ci acceptables en agriculture.
